# EUROPEAN PATENT APPLICATION

(11) **EP 4 134 366 A1**
(43) Date of publication of application: **15.02.2023**
(21) Application number: 21793225.0
(22) Date of filing: 19.04.2021
(51) Int. Cl.: C07D 401/14, A61K 31/439, A61K 31/4725, A61K 31/436, A61K 31/519, A61K 31/517, A61P 3/00, A61P 3/10, A61P 1/16, A61P 3/04

(54) **3-AZABICYCLOALKYL DERIVATIVE AND PHARMACEUTICAL COMPOSITION CONTAINING SAME**

(30) Priority: 20.04.2020 KR 20200047190
(71) Applicant: Lg Chem, Ltd., Seoul 07336 (KR)
(72) Inventor: LEE, Sung Bae, Daejeon 34122 (KR); YOUN, Suk Jun, Daejeon 34122 (KR); KIM, Chang Hoon, Daejeon 34122 (KR); MOON, Hee Jeong, Daejeon 34122 (KR)
(74) Representative: Hoffmann Eitle
(86) International application number: PCT/KR2021/004887
(87) International publication number: WO 2021/215765

(57) **Abstract**

The present invention relates to: a compound of chemical formula 1, a pharmaceutically acceptable salt thereof or an isomer thereof; and a pharmaceutical composition for treating or preventing metabolic diseases, containing same as an active ingredient. [Chemical formula 1] In chemical formula 1, R1 represents an unsubstituted or C1-C5 alkyl-substituted C3-C7 heterocycloalkyl containing one to three N atoms, R2 and R3 independently represent hydrogen, a halogen or a C1-C5 alkyl, R4 represents -(CH2)mCO2H, m, p and q are each independently an integer of 0-2, X represents N or C-CN, Y represents CH2 or O, and Z represents CH2 or a single bond.

## Description

### [Technical Field]

The present invention relates to a novel 3-azabicycloalkyl derivative compound having ketohexokinase (KHK) inhibitory activity and a pharmaceutical composition containing the same as an active ingredient.

### [Background Art]

It has been known that the causes of non-alcoholic steatohepatitis (NASH), which is one of the metabolic diseases, largely include the generation of fatty liver, increased inflammation and cell death. As a chronic disease, NASH may gradually develop to liver fibrosis, cirrhosis and liver cancer.

Meanwhile, ketohexokinase (KHK) is an enzyme involved in fructose metabolism, and is a type of kinase responsible for fructose phosphorylation in fructose metabolism. Unlike glucose metabolism, fructose metabolism induces rapid fat accumulation due to not being subject to energy-dependent inhibition, and affects fatty liver generation. Therefore, when KHK is inhibited, the effect of inhibiting the generation of fatty liver, which is one of the NASH causes, may be expected.

In an animal test model, when KHK activity was suppressed, it was observed that overall indicators for metabolic diseases are improved, the phenotype of a KHK-knock out mouse does not have a difference from a normal mouse, and thus it is expected that there will be no serious side effects due to KHK inhibition.

However, due to the fact that KHK is responsible for phosphorylation using ATP, since the suppression of KHK has the possibility of inhibiting a different type of kinase, there are many concerns about the safety of KHK used as a therapeutic agent for chronic diseases. Therefore, it is important that a KHK inhibitor has no selectivity for a different kinase.

Research on KHK inhibitors is currently being actively conducted, and it is still necessary to develop a therapeutic agent that can be effectively used in preventing or treating a metabolic disease such as diabetes, complications of diabetes, obesity, NASH, or steatohepatitis.

### [Related Art Document]

(Patent Document 1) U.S. Patent No. 9,809,579

### [Disclosure of Invention]

### [Technical Problem]

The present invention is directed to providing a novel compound having ketohexokinase (KHK) inhibitory activity, or a pharmaceutically acceptable salt or isomer thereof.

In addition, the present invention is directed to providing a pharmaceutical composition for preventing or treating a metabolic disease, which includes the above-described compound, or a pharmaceutically acceptable salt or isomer thereof.

In addition, the present invention is directed to providing a method of preventing or treating a metabolic disease, which includes administering the above-described compound, or a pharmaceutically acceptable salt or isomer thereof to a subject.

### [Solution to Problem]

The present invention provides a compound of Formula 1 below, or a pharmaceutically acceptable salt or isomer thereof. 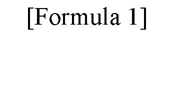 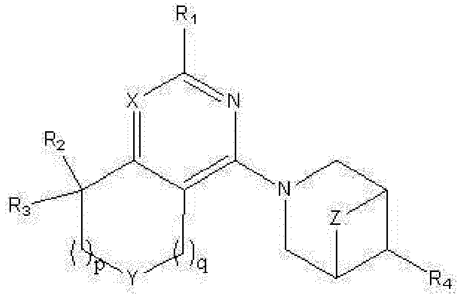 In Formula 1,
R₁ is an unsubstituted or C₁-C₅ alkyl-substituted C₃-C₇ heterocycloalkyl having one to three N atoms,
R₂ and R₃ are each independently hydrogen, halogen or a C₁-C₅ alkyl,
R₄ is -(CH₂)ₘCO₂H,
m, p and q are each independently an integer of 0 to 2,
X is N or C-CN,
Y is CH₂ or O, and
Z is CH₂ or a single bond.

In addition, the present invention provides a pharmaceutical composition for inhibiting KHK, which includes the above-described compound, or a pharmaceutically acceptable salt or isomer thereof; and a pharmaceutically acceptable carrier.

In addition, the present invention provides a pharmaceutical composition for preventing or treating a metabolic disease, which includes the above-described compound, or a pharmaceutically acceptable salt or isomer thereof; and a pharmaceutically acceptable carrier.

### [Detailed Description of the Invention]

Hereinafter, the present invention will be described in further detail to help in understanding the present invention. Here, terms and words used in the specification and claims should not be construed as limited to general or dictionary terms meanings, and should be interpreted with the meaning and concept in accordance with the technical idea of the present invention based on the principle that the inventors have appropriately defined the concepts of terms in order to describe the invention in the best way.

The present invention provides a compound of Formula 1 below, or a pharmaceutically acceptable salt or isomer thereof. 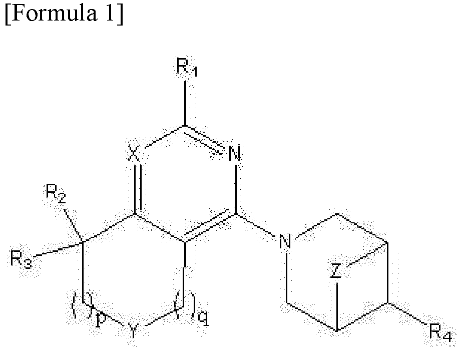 In Formula 1,
R₁ is unsubstituted or C₁-C₅ alkyl-substituted C₃-C₇ heterocycloalkyl having one to three N atoms,
R₂ and R₃ are each independently hydrogen, halogen or C₁-C₅ alkyl,
R₄ is -(CH₂)ₘCO₂H,
m, p and q are each independently an integer of 0 to 2,
X is N or C-CN,
Y is CH₂ or O, and
Z is CH₂ or a single bond.

Throughout the specification, to define the compound of Formula 1, the concepts defined as follows are used. Unless particularly indicated otherwise, the following definitions are also applied to terms used individually or as a part of a large group throughout the specification. Terms and abbreviations used herein have their original meanings unless defined otherwise.

The term "alkyl" refers to a linear or branched hydrocarbon radical, and preferably, a linear or branched saturated or unsaturated hydrocarbon radical having 1 to 5, 1 to 4, or 1 to 3 carbon atoms. In addition, each carbon atom of the alkyl may be optionally substituted with one or more halogens. For example, the alkyl substituted with alkyl or halogen may be methyl, ethyl, n-propyl, iso-propyl, n-butyl, tert-butyl, pentyl, fluoromethyl, trifluoromethyl, fluoroethyl or fluoropropyl, but the present invention is not limited thereto.

The term "heterocycloalkyl" refers to a partially or totally saturated hydrocarbon which includes one or more heteroatoms selected from N, O and S as a ring atom, and constitutes a single or fused cyclic ring. Preferably, there may be 1, 1 to 2, or 1 to 3 heteroatoms. Preferably, the heterocycloalkyl is C₃-C₇ heterocycloalkyl having one to three N atoms. For example, the heterocycloalkyl is azetidinyl, pyrrolidinyl, piperidinyl, morpholinyl, imidazolinyl or piperazinyl, but the present invention is not limited thereto.

The term "halogen" refers to a substituent selected from the group consisting of fluoro, chloro, bromo and iodo. Other terms and abbreviations used herein have their original meanings unless defined otherwise.

The compound of Formula 1 according to the present invention may form a pharmaceutically acceptable salt. The pharmaceutically acceptable salts include acid addition salts formed by an acid that forms a non-toxic acid addition salt containing a pharmaceutically acceptable anion, for example, an inorganic acid such as hydrochloric acid, sulfonic acid, nitric acid, phosphoric acid, hydrobromic acid or hydroionic acid, an organic acid such as tartaric acid, formic acid, citric acid, acetic acid, trichloroacetic acid, trifluoroacetic acid, gluconic acid, benzoic acid, lactic acid, fumaric acid, maleic acid or salicylic acid, or a sulfonic acid such as methanesulfonic acid, ethanesulfonic acid, benzenesulfonic acid or p-toluenesulfonic acid.

In addition, pharmaceutically acceptable carboxylic acid salts include, for example, an alkali metal or alkali earth metal salt formed by lithium, sodium, potassium, calcium or magnesium, an amino acid salt such as lysine, arginine or guanidine, and an organic salt such as dicyclohexylamine, N-methyl-D-glucamine, tris(hydroxymethyl) methylamine, diethanolamine, choline or triethylamine. The compound of Formula 1 according to the present invention may be converted to a salt thereof by a conventional method.

Since the compounds according to the present invention have an asymmetric carbon center and an asymmetric axis or asymmetric plane, they may be present as E or Z isomers, R or S isomers, racemates, a mixture of diastereoisomers, or an individual diastereoisomer, and all of the isomers and mixtures thereof are included in the scope of the present invention.

Hereinafter, unless indicated otherwise, for convenience, the compound of Formula 1 refers to all of the compound of Formula 1, a pharmaceutically acceptable salt thereof, and an isomer thereof.

Representative compounds of Formula 1 according to the present invention may include the following compounds, but the present invention is not limited thereto:
2-((1R,5S,6S)-3-(4-cyano-3-((S)-2-methylazetidin-1-yl)-6,7-dihydro-5H-cyclopenta[c]pyridin-1-yl)-3-azabicyclo[3.1.1]heptan-6-yl)aceticacid;
2-((1R,5S,6S)-3-(4-cyano-3-((S)-2-methylazetidin-1-yl)-5,6,7,8-tetrahydroisoquinolin-1-yl)-3-azabicyclo[3.1.1]heptan-6-yl)aceticacid;
2-(3-(4-cyano-3-((S)-2-methylazetidin-1-yl)-6,7,8,9-tetrahydro-5H-cyclohepta[c]pyridin-1-yl)-3-azabicyclo[3.1.1]heptan-6-yl)aceticacid;
2-((1R,5S,6S)-3-(5-cyano-6-((S)-2-methylazetidin-1-yl)-3,4-dihydro-1H-pyrano[3,4-c]pyridin-8-yl)-3-azabicyclo[3.1.1]heptan-6-yl)acetic acid;
2-(3-(7,7-dimethyl-2-((S)-2-methylazetidin-1-yl)-5,7-dihydrofuro[3,4-d]pyrimidin-4-yl)-3-azabicyclo[3.1.1]heptan-6-yl)acetic acid;
2-((1R,5 S,6S)-3-((S)-7-methyl-2-((S)-2-methylazetidin-1-yl)-5,7-dihydrofuro[3,4-d]pyrimidin-4-yl)-3-azabicyclo[3.1.1]heptan-6-yl)acetic acid;
2-((1R,5 S,6S)-3-((S)-7-methyl-2-((S)-2-methylazetidin-1-yl)-5,7-dihydrofuro[3,4-d]pyrimidin-4-yl)-3-azabicyclo[3.1.0]hexan-6-yl)acetic acid;
2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)acetic acid;
2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)acetic acid;
2-((1R,5S,6S)-3-(8,8-difluoro-2-((S)-2-methylazetidin-1-yl)-5,6,7,8-tetrahydroquinazolin-4-yl)-3-azabicyclo[3.1.1]heptan-6-yl)acetic acid;
2-((1R,5S,6S)-3-(8,8-difluoro-2-((S)-2-methylazetidin-1-yl)-5,6,7,8-tetrahydroquinazolin-4-yl)-3-azabicyclo[3.1.0]hexan-6-yl)acetic acid; and
2-((1R,5S,6S)-3-(8,8-difluoro-2-(pyrrolidin-1-yl)-5,6,7,8-tetrahydroquinazolin-4-yl)-3-azabicyclo[3.1.0]hexan-6-yl)acetic acid.

The compound of Formula 1 according to the present invention, or a pharmaceutically acceptable salt or isomer thereof has ketohexokinase (KHK) inhibitory activity.

The present invention provides a compound used as a KHK inhibitor, or a pharmaceutically acceptable salt or isomer thereof.

The present invention provides a compound used to prevent or treat a metabolic disease, particularly, a KHK-related metabolic disease, or a pharmaceutically acceptable salt or isomer thereof.

The compound of Formula 1 according to the present invention, or a pharmaceutically acceptable salt or isomer thereof is suitable for preventing or treating a KHK-related metabolic disease.

The present invention provides a pharmaceutical composition for a KHK inhibitor, which includes the compound of Formula 1, or a pharmaceutically acceptable salt or isomer thereof; and a pharmaceutically acceptable carrier.

In addition, various types of prodrugs that are *in vivo* converted to the compound of Formula 1 according to purpose are also included in the scope of the present invention.

The pharmaceutical composition according to the present invention may be used in preventing or treating a KHK-related metabolic disease. The KHK-related metabolic disease may be diabetes, complications of diabetes, obesity, non-alcoholic steatohepatitis (NASH) or steatohepatitis, but the present invention is not limited thereto. Preferably, the pharmaceutical composition according to the present invention may be used in preventing or treating NASH.

In addition, the present invention provides a method of preparing a pharmaceutical composition for preventing or treating a KHK-related metabolic disease, particularly, diabetes, complications of diabetes, obesity, NASH or steatohepatitis, which includes mixing the compound of Formula 1, or a pharmaceutically acceptable salt or isomer thereof, as an active ingredient, with a pharmaceutically acceptable carrier.

In addition, the present invention provides a use of the compound of Formula 1, or a pharmaceutically acceptable salt or isomer thereof to prepare a drug for treating a KHK-related metabolic disease. The KHK-related metabolic disease is diabetes, complications of diabetes, obesity, NASH or steatohepatitis, but the present invention is not limited thereto.

In addition, the "pharmaceutical composition" may include the compound of the present invention and other chemical components such as a diluent and a carrier. Therefore, a pharmaceutically acceptable carrier, diluent or excipient, or a combination thereof may be included in the pharmaceutical composition as needed. The pharmaceutical composition facilitates the administration of a compound into an organism. There are various techniques of administering a compound, which include oral administration, injections, administration of an aerosol, parenteral administration, and topical administration, but the present invention is not limited thereto.

The term "carrier" refers to a compound that facilitates the introduction of a compound into cells or tissue. For example, dimethyl sulfoxide (DMSO) is a conventional carrier that facilitates the introduction of many organic compounds into cells or tissue of an organism.

The term "diluent" is defined as a compound that not only stabilizes a biologically active form of a target compound, but also is diluted in water dissolving the compound. Salts dissolved in a buffer are used as a diluent in the art. A commonly used buffer is phosphate buffered saline that mimics the salt form of a human solution. Since the buffered salt can control the pH of a solution at a low concentration, a buffered diluent rarely changes the biological activity of a compound.

The term "pharmaceutically acceptable" used herein refers to a property of not impairing the biological activity and physical properties of a compound.

The compound of the present invention may be formulated in various pharmaceutical dosage forms according to purpose. When the pharmaceutical composition according to the present invention is prepared, active ingredients, specifically, the compound of Formula 1, or a pharmaceutically acceptable salt or isomer thereof is mixed with various pharmaceutically acceptable carriers which may be selected according to a formulation to be prepared. For example, the pharmaceutical composition according to the present invention may be formulated into an injectable preparation or an oral preparation according to purpose.

The compound of the present invention may be prepared by a known method using known pharmaceutical carriers and excipients and may be contained in a unit dose form or multi-dose container. The form of a preparation may be a solution, suspension or emulsion in an oil or aqueous medium, and may contain a conventional dispersing, suspending or stabilizing agent. In addition, for example, the preparation may be formed as a dry powder used by being dissolved in sterile pyrogen-free water before use. The compound of the present invention may also be prepared as a suppository using a conventional suppository base such as cocoa butter or other glycerides. The solid dosage form for oral administration may be a capsule, a tablet, a pill, powder or granule, and particularly, a capsule and a tablet are useful. The tablet and pill are preferably prepared with an enteric coating agent. The solid dosage form may be prepared by mixing the compound of the present invention with one or more inert diluents such as sucrose, lactose and starch, and carriers such as a lubricant such as magnesium stearate, a disintegrant and a binder. As needed, the compound according to the present invention or a pharmaceutical composition containing the same may be administered in combination with other drugs, for example, other therapeutic agents for diabetes.

In addition, the present invention provides a method of preventing or treating a metabolic disease, which includes administering the compound of Formula 1, or a pharmaceutically acceptable salt or isomer thereof; or a pharmaceutical composition including the same to a subject.

The subject may be a human or a mammal excluding a human, which requires treatment or prevention of a metabolic disease, and the metabolic disease may be a KHK-related metabolic disease. Representative examples of the metabolic diseases may include diabetes, complications of diabetes, obesity, NASH and steatohepatitis, but the present invention is not limited, and preferably, NASH.

The term "treatment" used herein means to stop, delay or alleviate the progression of a disease when a drug is used in a subject showing symptoms of a disease, and the "prevention" means to stop, delay or alleviate the onset of a disease when a drug is used in a subject showing no symptoms of the disease, but is at high risk of the onset of a disease

A dosage of the compound of Formula 1 of the present invention, or a pharmaceutically acceptable salt or isomer thereof is determined by a doctor's prescription depending on factors such as a patient's body weight, age and the specific nature and severity of a disease. However, a dosage required for adult treatment is usually in the range of approximately 1 to 500 mg per day, according to the frequency and intensity of administration. When administered intramuscularly or intravenously to an adult, it is sufficient that a total dosage of approximately 1 to 300 mg of the compound of Formula 1 according to the present invention is generally administered per day with divided daily doses, but for some patients, higher daily doses may be preferable.

The present invention also provides a method of preparing a compound of Formula 1. Hereinafter, to help in understanding of the present invention, the method of preparing the compound of Formula 1 will be described based on exemplary reaction schemes. However, the compound of Formula 1 may be prepared by various methods based on the structure of Formula 1 by one of ordinary skill in the art to which the present invention belongs, and these methods should be construed as being included in the scope of the present invention. That is, the compound of Formula 1 may be prepared by arbitrarily combining various synthesis methods described in the specification or disclosed in the related art, which will be understood as being included in the scope of the present invention, and the method of preparing the compound of Formula 1 is not limited to the following description.

When the compound of the present invention is prepared, a reaction sequence may be properly changed. That is, one process may be previously performed or a process of changing any substituent may be inserted, and as needed, any other reagents other than the exemplified reagents may be used. A compound obtained in each process may be separated or purified by a conventional method such as recrystallization, distillation or a silica gel column. In addition, a subsequent process may be performed without separation or purification of a compound obtained in each process.

In the reaction schemes below, unless defined otherwise, all substituents are the same as defined above. Reagents and starting materials may be easily obtained commercially. The others may be prepared by synthesis methods to be described in the following preparation examples and examples as well as a synthesis method for a known compound which is structurally similar to that of the present invention. Unless a preparation method is particularly described, a compound used as a starting material is a known compound, or a compound which may be synthesized from a known compound by a known synthesis method or a similar method thereto.

Hereinafter, the present invention will be described in further detail with reference to preparation examples and examples, but the scope of the present invention is not limited thereto.

### [Preparation Example 1] Preparation of 1,3-dichloro-6,7-dihydro-5H-cyclopenta[c]pyridine-4-carbonitrile

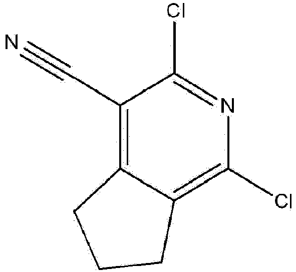

A compound of Preparation Example 1 was prepared through the following Steps A and B.

### (Step A) Preparation of 1,3-dihydroxy-6,7-dihydro-5H-cyclopenta[c]pyridine-4-carbonitrile

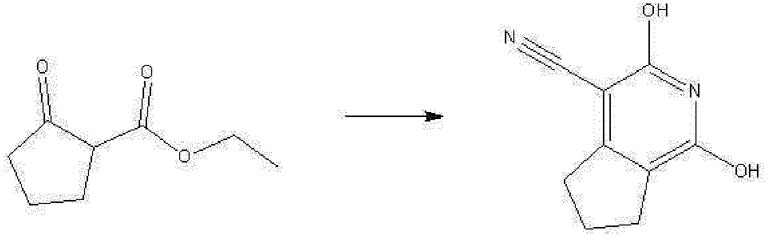

Ethyl 2-oxocyclopentane-1-carboxylate (4.0 g, 25.6 mmol), 2-cyanoacetamide (2.15 g, 25.6 mmol) and potassium hydroxide (1.58 g, 28.2 mmol) were dissolved in methanol (100 ml), and stirred under reflux for 12 hours. The reaction solution was concentrated under reduced pressure, and an 1N hydrochloric acid solution was added. The generated solid was filtered, washed with water and dried with nitrogen, thereby obtaining 1,3-dihydroxy-6,7-dihydro-5H-cyclopenta[c]pyridine-4-carbonitrile (1.5 g, 8.51 mmol, 33%).

### (Step B) Preparation of 1,3-dichloro-6,7-dihydro-5H-cyclopenta[c]pyridine-4-carbonitrile

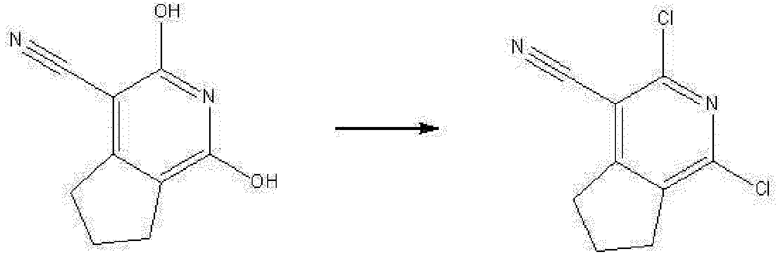

The 1,3-dihydroxy-6,7-dihydro-5H-cyclopenta[c]pyridine-4-carbonitrile (1.5 g, 8.51 mmol) obtained in Step A, benzyltriethylammonium chloride (3.6 g, 15.9 mmol) and phosphoryl trichloride (3.7 ml, 39.7 mmol) were reacted for 5 hours at a reaction temperature of 100 °C. The reaction solution was concentrated under reduced pressure, and water was added, followed by extraction with ethyl acetate. An organic layer was washed with an aqueous sodium chloride solution, an aqueous sodium hydrogen carbonate solution and water, dried with anhydrous magnesium sulfate, and filtered. The filtrate was concentrated under reduced pressure and purified by column chromatography (ethyl acetate/hexane = 1/5), thereby obtaining 1,3-dichloro-6,7-dihydro-5H-cyclopenta[c]pyridine-4-carbonitrile (1.0 g, 4.69 mmol, 55%).

1H-NMR (500MHz, CDCl₃) δ 3.23 (t, J = 7.8 Hz, 2H), 3.06 (t, J = 7.6 Hz, 2H), 2.30 (t, J = 7.6 Hz, 2H)

### [Preparation Example 2] Preparation of 1,3-dichloro-5,6,7,8-tetrahydroisoquinoline-4-carbonitrile

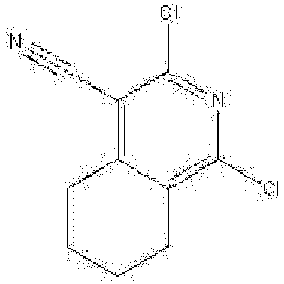

By the method described in Preparation Example 1, 1,3-dichloro-5,6,7,8-tetrahydroisoquinoline-4-carbonitrile (1.0 g, 4.40 mmol, 15%) was obtained using ethyl 2-oxocyclohexane-1-carboxylate (5.0 g, 29.4 mmol) instead of ethyl 2-oxocyclopentane-1 -carboxylate.

1H-NMR (500MHz, CDCl₃) δ 2.99 (d, J = 5.6 Hz, 2H), 2.77 (t, J = 5.6 Hz, 2H), 1.89 (d, J = 5.5 Hz, 4H)

### [Preparation Example 3] Preparation of 1,3-dichloro-6,7,8,9-tetrahydro-SH-cyclohepta[c]pyridine-4-carbonitrile

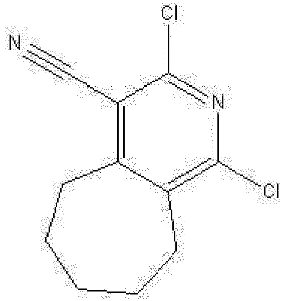

By the method described in Preparation Example 1, 1,3-dichloro-6,7,8,9-tetrahydro-5H-cyclohepta[c]pyridine-4-carbonitrile (660 mg, 2.74 mmol, 23%) was obtained using methyl 2-oxocycloheptane-1-carboxylate (2.0 g, 11.8 mmol) instead of ethyl 2-oxocyclopentane-1-carboxylate.

1H-NMR (500MHz, CDCl₃): δ3.15 (t, J = 5.5 Hz, 2H), 3.08 (t, J = 5.5 Hz, 2H), 1.92 (q, J = 5.9 Hz, 2H), 1.83-1.72 (m, 2H), 1.72-1.61 (m, 2H)

### [Preparation Example 4] Preparation of 6,8-dichloro-3,4-dihydro-1H-pyrano[3,4-c]pyridine-5-carbonitrile

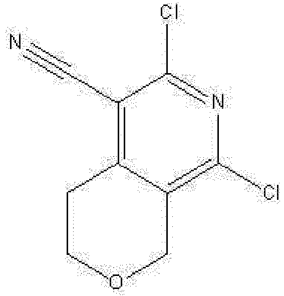

By the method described in Preparation Example 1, 6,8-dichloro-3,4-dihydro-1H-pyrano[3,4-c]pyridine-5-carbonitrile (200 mg, 0.87 mmol, 7.5%) was obtained using ethyl 4-oxotetrahydro-2H-pyrane-3-carboxylate (2.0 g, 11.6 mmol) instead of ethyl 2-oxocyclopentane-1-carboxylate.

1H-NMR (500MHz, CDCl₃) δ 4.73 (s, 2H), 4.08-3.96 (2H), 3.06 (t, J = 5.5 Hz, 2H)

### [Preparation Example 5] Preparation of (S)-2,4-dichloro-7-methyl-5,7-dihydrofuro[3,4-d]pyrimidine

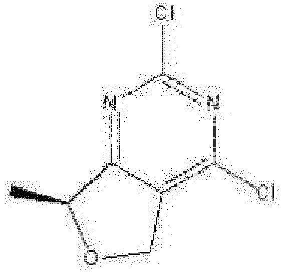

A compound of Preparation Example 5 was prepared through the following steps A, B, C and D.

### (Step A) Preparation of methyl (5S)-5-methyl-4-oxotetrahydrofuran-3-carboxylate

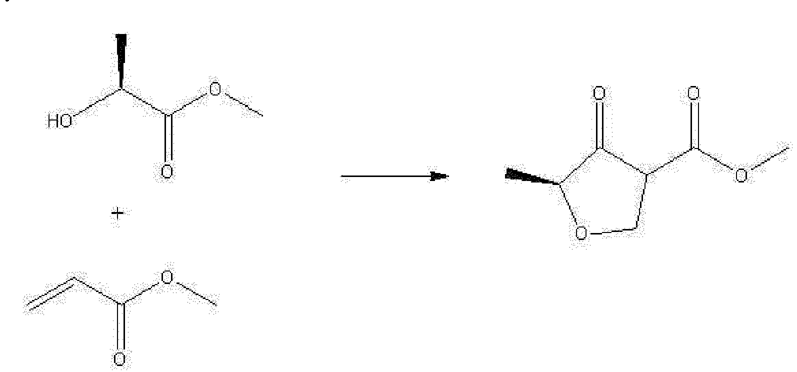

Methyl (S)-2-hydroxypropionate (42.0 g, 38.4 mmol) and sodium hydride (1.54 g, 60%, 38.4 mmol) were dissolved in tetrahydrofuran (100 ml) and stirred at 0 °C for 30 minutes, followed by concentration under reduced pressure.

Methyl acrylate (3.46 ml, 38.4 mmol) and dimethyl sulfoxide (30 ml) were added to the concentrated material and stirred at room temperature for 12 hours, and thus the reaction was completed. A 1N hydrochloric solution was added to the resulting reaction solution, and extraction was performed with ethyl acetate. The resulting extract was washed with saturated sodium chloride, dried with anhydrous magnesium sulfate and filtered, and then the resulting filtrate was distilled under reduced pressure. The resulting product was purified by column chromatography (ethyl acetate/hexane = 1/5), thereby obtaining methyl (5S)-5-methyl-4-oxotetrahydrofuran-3-carboxylate (2.0 g, 12.7 mmol, 33%).

1H-NMR (400MHz, CDCl₃) δ 4.61-4.56 (0.3H), 4.52 (d, J = 9.2 Hz, 0.7H), 4.38-4.23 (m, 1H), 3.98 (d, J = 6.7 Hz, 0.3H), 3.91 (d, J = 7.0 Hz, 0.7H), 3.85-3.74 (3H), 3.62-3.47 (m, 1H), 1.35 (dd, J = 6.9, 2.3 Hz, 3H)

### (Step B) Preparation of methyl (S)-5-methyl-4-ureido-2,5-dihydrofuran-3-carboxylate

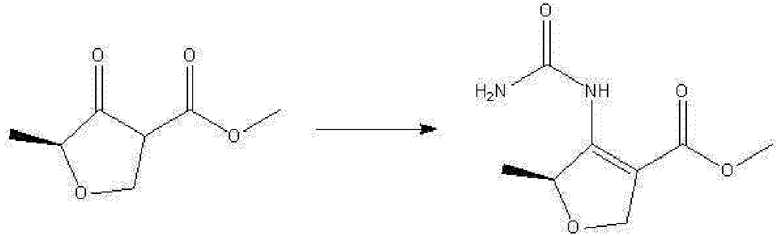

The methyl (5S)-5-methyl-4-oxotetrahydrofuran-3-carboxylate obtained in Step A (4.0 g, 25.3 mmol), urea (7.6 g, 126 mmol) and concentrated sulfuric acid (1.35 ml, 25.3 mmol) were dissolved in methanol (100 ml), and stirred under reflux for 8 hours. The resulting reaction solution was concentrated under reduced pressure, and an excessive amount of water was added to precipitate a solid. Then, the precipitated solid was filtered and then dried with nitrogen, thereby obtaining methyl (S)-5-methyl-4-ureido-2,5-dihydrofuran-3-carboxylate (2.0 g, 9.99 mmol, 40%).

1H-NMR (400MHz, DMSO-D6) δ9.19-9.03 (m, 1H), 6.97 (s, 2H), 5.59 (t, J = 5.2 Hz, 1H), 4.66-4.44 (m, 2H), 3.68 (d, J = 6.4 Hz, 3H), 2.51 (s, 1H), 1.37-1.15 (3H)

### (Step C) Preparation of (S)-7-methyl-5,7-dihydrofuro[3,4-d]pyrimidine-2,4-diol

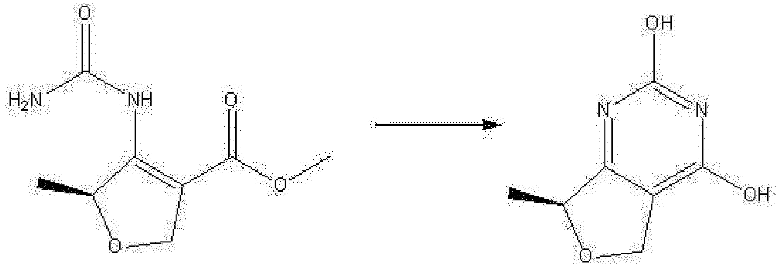

The methyl (S)-5-methyl-4-ureido-2,5-dihydrofuran-3-carboxylate (2.0 g, 9.99 mmol) obtained in Step B was dissolved in 28% ammonia water (20 ml), and stirred at 60 °C for 12 hours. After the reaction was completed, concentration under reduced pressure was performed. A 1N hydrochloric acid solution was added to the concentrated material, and extraction was performed with ethyl acetate. The resulting extract was washed with saturated sodium chloride, dried with anhydrous magnesium sulfate and then filtered, and the resulting filtrate was distilled under reduced pressure. The resulting product was purified by column chromatography (methanol/dichloromethane = 1/9), thereby obtaining (S)-7-methyl-5,7-dihydrofuro[3,4-d]pyrimidine-2,4-diol (1.0 g, 5.95 mmol, 60%).

1H-NMR (400MHz, DMSO-D6) δ 11.40 (s, 1H), 11.02 (s, 1H), 5.06-4.93 (m, 1H), 4.78-4.67 (1H), 4.63 (dd, J = 10.5, 2.6 Hz, 1H), 3.32 (s, 2H), 2.57-2.45 (4H), 1.36 (d, J = 6.4 Hz, 4H)

### (Step D) Preparation of (S)-2,4-dichloro-7-methyl-5,7-dihydrofuro[3,4-d]pyrimidine

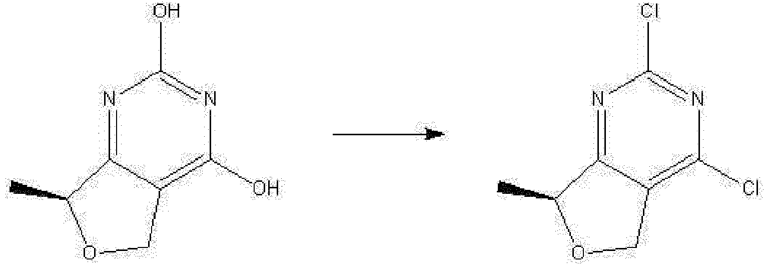

The (S)-7-methyl-5,7-dihydrofuro[3,4-d]pyrimidine-2,4-diol obtained in Step C (1.0 g, 5.95 mmol), benzyl triethylammonium chloride (1.4 g, 5.95 mmol) and phosphoryl trichloride (8.3 ml) were dissolved in dichloroethane (20 ml), and reacted at 100 °C for 8 hours. After the reaction solution was concentrated under reduced pressure, water was added, followed by extraction with ethyl acetate. An organic layer was washed with an aqueous saturated sodium chloride solution, an aqueous sodium hydrogen carbonate solution and water, dried with anhydrous magnesium sulfate, and filtered. The filtrate was concentrated under reduced pressure, and purified by column chromatography (ethyl acetate/hexane = 1/5), thereby obtaining (S)-2,4-dichloro-7-methyl-5,7-dihydrofuro[3,4-d]pyrimidine (1.0 g, 4.88 mmol, 82%).

1H-NMR (400MHz, CDCl₃) δ5.25 (td, J = 4.4, 2.2 Hz, 1H), 5.12 (ddd, J = 32.0, 13.7, 2.1 Hz, 2H), 1.58 (d, J = 6.4 Hz, 3H)

### [Preparation Example 6] 4-chloro-7,7-difluoro-2-(methylthio)-6,7-dihydro-5H-cyclopenta[d]pyrimidine

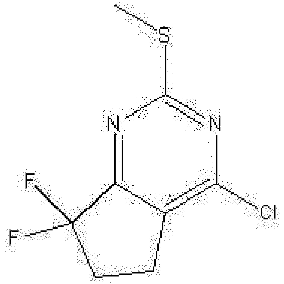

A compound of Preparation Example 6 was prepared through the following Steps A, B, C, D, E, and F.

### (Step A) Preparation of 6-ethoxy-5,6-dioxohexanoic acid

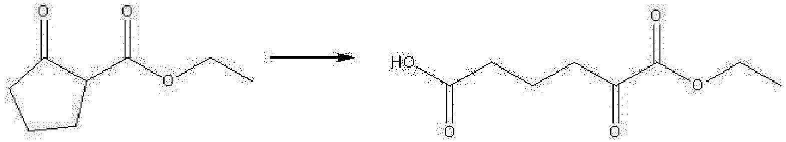

Ethyl 2-oxocyclopentane-1-carboxylate (4.0 g, 25.6 mmol) and sodium hydride (1.0 g, 60%, 25.6 mmol) were dissolved in tetrahydrofuran (100 ml), and stirred at 0 °C for 30 minutes, followed by concentration under reduced pressure. Nitrosobenzene (2.88 g, 26.9 mmol) was added to the reaction solution and stirred at 0 °C for 30 minutes, and therefore, the reaction was completed. A 1N hydrochloric acid solution was added to the resulting reaction solution, and extraction was performed with ethyl acetate. The resulting extract was washed with an aqueous saturated sodium chloride solution and dried with anhydrous magnesium sulfate, and the resulting filtrate was distilled under reduced pressure, thereby obtaining 6-ethoxy-5,6-dioxohexanoic acid (3.6 g, 19.1 mmol). The obtained material was used in the following reaction without purification.

1H-NMR (400MHz, CDCl₃) δ4.31(q, J=7.2Hz, 2H), 2.94(t, J=7.1Hz, 2H), 1.96(t, J=6.8Hz, 2H), 1.36(t, J=7.2Hz, 2H)

### (Step B) Preparation of 1-ethyl 6-methyl 2-oxohexanedioate

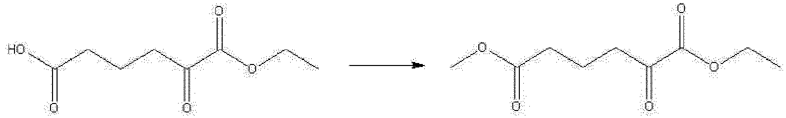

The 6-ethoxy-5,6-dioxohexanoic acid obtained in Step A (3.6 g, 19.1 mmol) was dissolved in ether (100 ml), and a 0.15M diazomethane ether solution (127 ml, 19.1 mmol) was slowly added dropwise at 0 °C. The resulting reaction solution was concentrated under reduced pressure, and purified by column chromatography (ethyl acetate/hexane = 1/2), thereby obtaining 1-ethyl-6-methyl 2-oxohexanedioate (2.7 g, 13.4 mmol, 52%, 2-step yield).

1H-NMR (500MHz, CDCl₃) δ4.34(q, 2H), 3.69(s, 3H), 2.95(t, 2H), 2.41(t, 2H), 1.99(m, 2H), 1.39(t, 3H)

### (Step C) Preparation of 1-ethyl-6-methyl 2,2-difluorohexanedioate

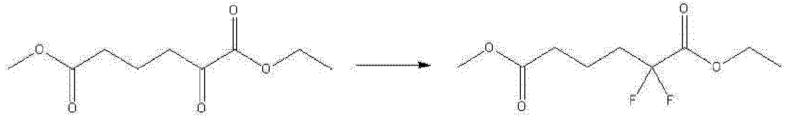

The 1-ethyl-6-methyl 2-oxohexanedioate obtained in Step B (2.8 g, 13.4 mmol) and diethylaminosulfur trifluoride (6.7 g, 41.5 mmol) were dissolved in chloroform (150 ml) and stirred at 0 °C for 3 hours. An excessive amount of water was added to the resulting reaction solution, and extraction was performed with ethyl acetate. An organic layer was washed with an aqueous sodium chloride solution, an aqueous sodium hydrogen carbonate solution and water, dried with anhydrous magnesium sulfate, and filtered. The filtrate was concentrated under reduced pressure and purified by column chromatography (ethyl acetate/hexane = 1/10), thereby obtaining 1-ethyl-6-methyl 2,2-difluorohexanedioate (0.9 g, 4.01 mmol, 30%).

1H-NMR (500MHz, CDCl₃) δ4.35(q, 2H), 3.69(s, 3H), 2.42(m, 2H), 2.14(m, 2H), 1.86(m, 2H), 1.38(t, 3H)

### (Step D) Preparation of methyl 2-ethoxy-3,3-difluoro-2-hydroxycyclopentane-1-carboxylate

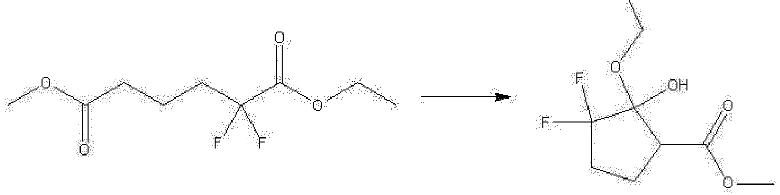

The 1-ethyl-6-methyl 2,2-difluorohexanedioate obtained in Step C (0.9 g, 4.01 mmol) and potassium tert-butoxide (473 mg, 4.21 mmol) were dissolved in tetrahydrofuran (100 ml), and stirred at room temperature for 8 hours. A 1N hydrochloric acid solution was added to the resulting reaction solution, and extraction was performed with ethyl acetate. An organic layer was washed with an aqueous sodium chloride solution, an aqueous sodium hydrogen carbonate solution and water, dried with aqueous magnesium sulfate and filtered. The filtrate was concentrated under reduced pressure, thereby obtaining methyl 2-ethoxy-3,3-difluoro-2-hydroxycyclopentane-1-carboxylate. The obtained material was used in a subsequent reaction without purification.

### (Step E) Preparation of 7,7-difluoro-2-(methylthio)-6,7-dihydro-5H-cyclopenta[d]pyrimidin-4-ol

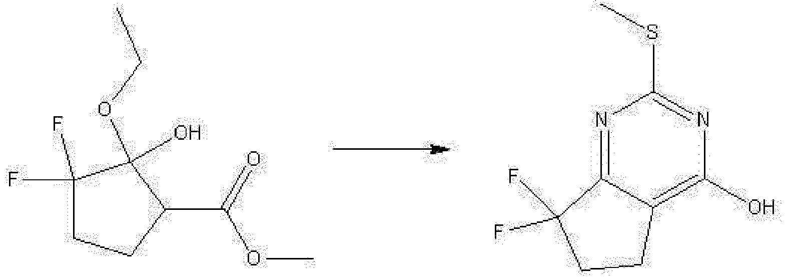

The methyl 2-ethoxy-3,3-difluoro-2-hydroxycyclopentane-1-carboxylate obtained in Step D (540 mg, 2.41 mmol), methylisothiourea hemisulfate (672 mg, 4.82 mmol) and sodium carbonate (767 mg, 7.24 mmol) were dissolved in water (50 ml), and stirred at room temperature for 12 hours. An excessive amount of water was added to the resulting reaction solution, and extraction was performed with ethyl acetate. An organic layer was washed with an aqueous sodium chloride solution, dried with an anhydrous magnesium sulfate, and filtered. The filtrate was concentrated under reduced pressure, and purified by column chromatography (ethyl acetate/hexane = 1/1), thereby obtaining 7,7-difluoro-2-(methylthio)-6,7-dihydro-5H-cyclopenta[d]pyrimidin-4-ol (250 mg, 1.15 mmol, 48%).

1H-NMR (400MHz, CDCl₃) δ10.92 (s, 1H), 2.84 (m, 2H), 2.68 (s, 3H), 2.50-2.63 (m, 2H)

### (Step F) Preparation of 4-chloro-7,7-difluoro-2-(methylthio)-6,7-dihydro-5H-cyclopenta[d]pyrimidine

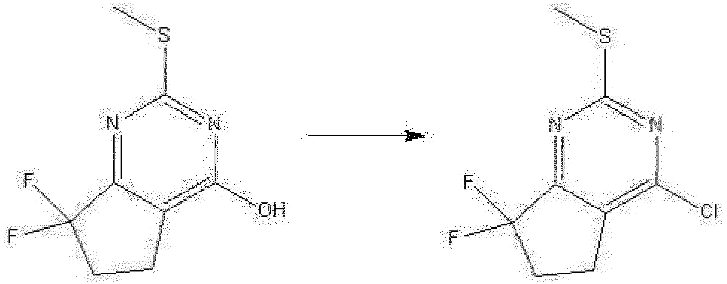

The 7,7-difluoro-2-(methylthio)-6,7-dihydro-5H-cyclopenta[d]pyrimidin-4-ol obtained in Step E (200 mg, 1.15 mmol), phosphoryl trichloride (1.1 ml, 11.5 mmol) and triethylamine (0.3 ml, 2.3 mmol) were dissolved in dichloroethane (30 ml), and reacted at 120 °C for 2 hours. After the reaction solution was concentrated under reduced pressure, water was added, and extraction was performed with ethyl acetate. An organic layer was washed with an aqueous saturated sodium chloride solution, dried with anhydrous magnesium sulfate, and filtered. The filtrate was concentrated under reduced pressure, and purified by column chromatography (methanol/dichloromethane = 1/19), thereby obtaining 4-chloro-7,7-difluoro-2-(methylthio)-6,7-dihydro-5H-cyclopenta[d]pyrimidine (130 mg, 0.55 mmol, 48%).

1H-NMR (400MHz, CDCl₃) δ2.95-3.00(m, 2H), 2.58-2.68(m, 2H), 2.65(s, 3H)

### [Preparation Example 7] Preparation of 4-chloro-8,8-difluoro-2-(methylthio)-5,6,7,8-tetrahydroquinazoline

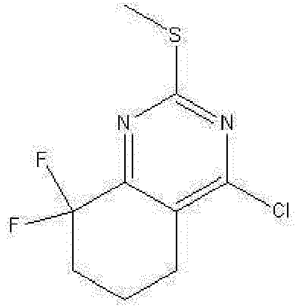

A compound of Preparation Example 7 was prepared through the following procedures.

By the methods described in Steps A to E of Preparation Example 6, 8,8-difluoro-2-(methylthio)-5,6,7,8-tetrahydroquinazolin-4-ol (6.25 g, 26.9 mmol, 23%, 4 steps) was obtained using ethyl 2-oxocyclohexane-1-carboxylate (20.0 g, 117.5 mmol) instead of ethyl 2-oxocyclopentane-1-carboxylate.

8,8-difluoro-2-(methylthio)-5,6,7,8-tetrahydroquinazolin-4-ol (3.0 g, 12.9 mmol), phosphoryl trichloride (12.8 ml, 129.2 mmol) and triethylamine (0.89 ml, 6.45 mmol) were dissolved in dichloroethane (200 ml), and reacted at 100 °C for 1 hour. The reaction solution was concentrated under reduced pressure, water was added, and extraction was performed with ethyl acetate. An organic layer was washed with an aqueous saturated sodium chloride solution, dried with anhydrous magnesium sulfate and filtrated. The filtrate was concentrated under reduced pressure, and purified by column chromatography (hexane/ethyl acetate = 2/1), thereby obtaining 4-chloro-8,8-difluoro-2-(methylthio)-5,6,7,8-tetrahydroquinazoline (2.43 g, 9.72 mmol, 75%).

1H-NMR (500MHz, CDCl₃) δ2.85-2.71 (m, 2H), 2.60 (s, 3H), 2.43-2.24 (m, 2H), 2.04 (tt, J = 9.4, 3.2 Hz, 2H)

The NMR results for intermediates related to the synthesis of Preparation Example 7 was obtained as follows:

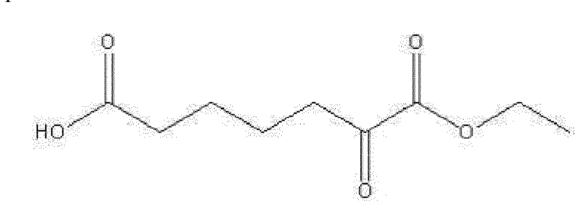

7-ethoxy-6,7-dioxoheptanoic acid: 1H-NMR (400MHz, CDCl₃) δ 4.40-4.25 (m, 2H), 3.49 (t, J= 1.5 Hz, 1H), 2.87 (t, J = 6.1 Hz, 2H), 2.46-2.32 (m, 2H), 1.77-1.61 (m, 4H), 1.44-1.30 (m, 3H)

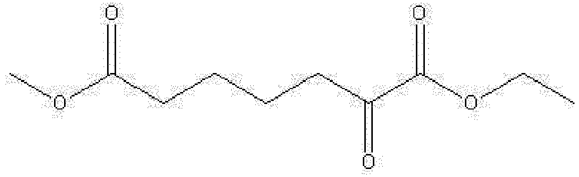

1-ethyl 7-methyl-2-oxoheptanedioate: 1H-NMR (400MHz, CDCl₃) δ 4.33 (qd, J = 7.1, 1.0 Hz, 2H), 3.68 (d, J = 1.2 Hz, 3H), 2.88 (d, J = 5.2 Hz, 2H), 2.36 (s, 2H), 1.76-1.61 (5H), 1.38 (td, J = 7.1, 1.0 Hz, 3H)

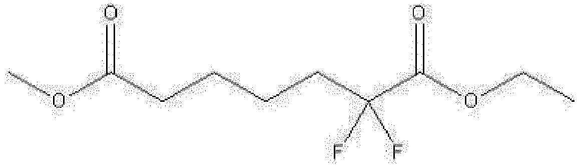

1-ethyl 7-methyl 2,2-difluoroheptanedioate: 1H-NMR (400MHz, CDCl₃) δ 4.34 (q, J = 7.1 Hz, 2H), 3.69 (s, 3H), 2.35 (t, J = 7.5 Hz, 2H), 2.09 (t, J = 8.2 Hz, 2H), 1.71 (t, J = 7.6 Hz, 2H), 1.53 (t, J = 8.1 Hz, 2H), 1.37 (t, J = 7.0 Hz, 3H)

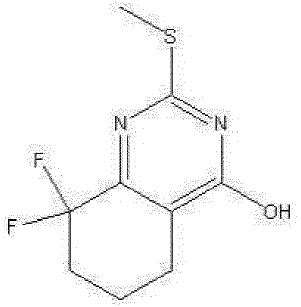

8,8-difluoro-2-(methylthio)-5,6,7,8-tetrahydroquinazolin-4-ol: 1H-NMR (400MHz, DMSO-D6) δ 2.50 (s, 3H), 2.46-2.35 (2H), 2.23 (t, J = 6.4 Hz, 2H), 1.80 (t, J = 6.3 Hz, 2H)

### [Example 1]

### 2-((1R,5S,6S)-3-(4-cyano-3-((S)-2-methylazetidin-1-yl)-6,7-dihydro-5H-cyclopenta[c]pyridin-1-yl)-3-azabicyclo[3.1.1]heptan-6-yl)acetic acid

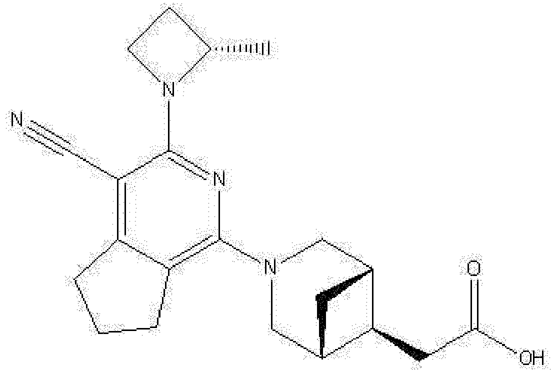

The compound of Example 1 was prepared through the following Steps A, B and C.

### (Step A) Preparation of ethyl 2-(3-(3-chloro-4-cyano-6,7-dihydro-5H-cyclopenta[c]pyridin-1-yl)-3-azabicyclo[3.1.1]heptan-6-yl)acetate

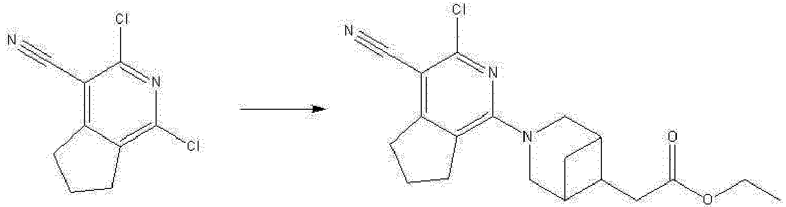

The 1,3-dichloro-6,7-dihydroxy-5H-cyclopenta[c]pyridine-4-carbonitrile obtained in Preparation Example 1 (100 mg, 0.47 mmol), ethyl 2-(3-azabicyclo[3.1.1]heptan-6-yl)acetate hydrochloride (97 mg, 0.47 mmol) and diisopropylethyl amine (0.33 ml, 1.88 mmol) were dissolved in dioxane (10 ml), and stirred at 50 °C for 8 hours. After the reaction was completed, a 1N hydrochloric acid aqueous solution was added, and extraction was performed with acetate. An organic layer was washed with an aqueous sodium chloride solution, an aqueous sodium hydrogen carbonate solution and water, dried with anhydrous magnesium sulfate and filtered. The filtrate was concentrated under reduced pressure, and purified by column chromatography (ethyl acetate/hexane = 1/5), thereby obtaining ethyl 2-(3-(3-chloro-4-cyano-6,7-dihydro-5H-cyclopenta[c]pyridin-1-yl)-3-azabicyclo[3.1.1]heptan-6-yl)acetate (60 mg, 0.17 mmol, 37%).

### (Step B) Preparation of ethyl 2-(3-(4-cyano-3-((S)-2-methylazetidin-1-yl)-6,7-dihydro-5H-cyclopenta[c]pyridin-1-yl)-3-azabicyclo[3.1.1]heptan-6-yl)acetate

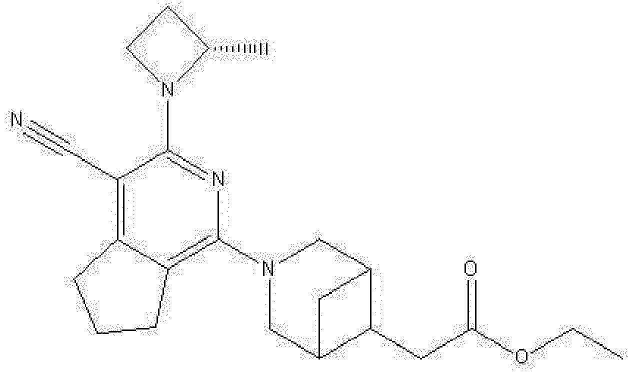

The ethyl 2-(3-(3-chloro-4-cyano-6,7-dihydro-5H-cyclopenta[c]pyridin-1-yl)-3-azabicyclo[3.1.1]heptan-6-yl)acetate obtained in Step A (60 mg, 0.17 mmol), (S)-2-methylazetidine hydrochloride (27 mg, 0.26 mmol) and potassium carbonate (94 mg, 0.68 mmol) were dissolved in N-methylprolidine (4 ml), and stirred at 100 °C for 12 hours. After the reaction was completed, an 1N hydrochloric acid aqueous solution was added, followed by extraction with acetate. An organic layer was washed with an aqueous sodium chloride solution, an aqueous sodium hydrogen carbonate solution and water, dried with anhydrous magnesium sulfate, and filtered. The filtrate was concentrated under reduced pressure and purified by column chromatography (ethyl acetate/hexane = 1/5), thereby obtaining ethyl 2-(3-(4-cyano-3-((S)-2-methylazetidin-1-yl)-6,7-dihydro-SH-cyclopenta[c]pyridin-1-yl)-3-azabicyclo[3.1.1]heptan-6-yl)acetate (35 mg, 0.092 mmol, 54%).

### (Step C) Preparation of 2-((1R,SS,6S)-3-(4-cyano-3-((S)-2-methylazetidin-1-yl)-6,7-dihydro-5H-cyclopenta[c]pyridin-1-yl)-3-azabicyclo[3.1.1]heptan-6-yl)acetic acid

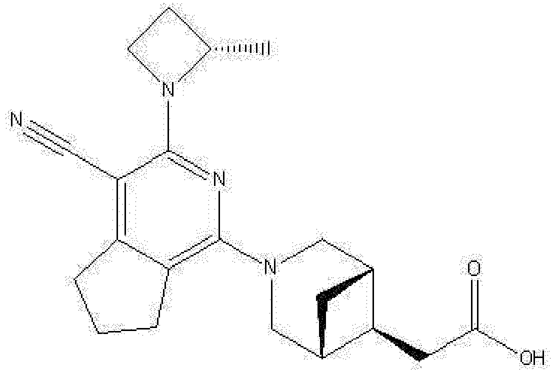

The ethyl 2-(3-(4-cyano-3-((S)-2-methylazetidin-1-yl)-6,7-dihydro-5H-cyclopenta[c]pyridin-1-yl)-3-azabicyclo[3.1.1]heptan-6-yl)acetate obtained in Step B (35 mg, 0.092 mmol) and lithium hydroxide monohydrate (15 mg, 0.37 mmol) were dissolved in a 20% tetrahydrofuran solution (5 ml), and stirred at room temperature for 10 hours. After the reaction was completed, an aqueous 1N hydrochloric solution was added, followed by extraction with acetate. An organic solution was washed with an aqueous sodium chloride solution, dried with anhydrous magnesium sulfate and filtered. The filtrate was concentrated under reduced pressure, and purified by column chromatography (ethyl acetate/hexane = 1/2), thereby obtaining 2-((1R,5S,6S)-3-(4-cyano-3-((S)-2-methylazetidin-1-yl)-6,7-dihydro-5H-cyclopenta[c]pyridin-1-yl)-3-azabicyclo[3.1.1]heptan-6-yl)acetic acid (10 mg, 0.027 mmol, 29%, relatively more polar compound) and 2-((1R,5S,6R)-3-(4-cyano-3-((S)-2-methylazetidin-1-yl)-6,7-dihydro-5H-cyclopenta[c]pyridin-1-yl)-3-azabicyclo[3.1.1]heptan-6-yl)acetic acid (relatively less polar compound).

1H-NMR (500MHz, CDCl₃) δ 4.58 (q, J = 6.7 Hz, 1H), 4.41 (td, J = 8.4, 4.5 Hz, 1H), 4.15-3.86 (m, 5H), 3.24-3.10 (2H), 2.85 (t, J = 7.6 Hz, 2H), 2.73 (d, J = 7.9 Hz, 2H), 2.48-2.28 (m, 4H), 2.14 (dd, J = 13.1, 7.6 Hz, 1H), 2.07-1.93 (m, 3H), 1.58-1.39 (m, 4H)

### [Example 2]

### 2-((1R,SS,6S)-3-(4-cyano-3-((S)-2-methylazetidin-1-yl)-5,6,7,8-tetrahydroisoquinolin-1-yl)-3-azabicyclo[3.1.1]heptan-6-yl)acetic acid

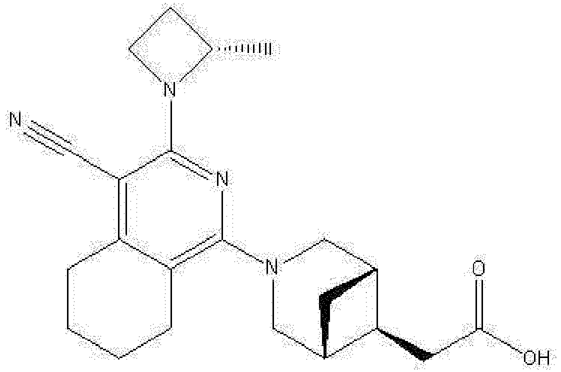

By the method described in Example 1, 2-((1R,5S,6S)-3-(4-cyano-3-((S)-2-methylazetidin-1-yl)-5,6,7,8-tetrahydroisoquinolin-1-yl)-3-azabicyclo[3.1.1]heptan-6-yl)acetic acid (11 mg, 0.029 mmol 7%, 3 steps) was obtained using the 1,3-dichloro-5,6,7,8-tetrahydroisoquinoline-4-carbonitrile obtained in Preparation Example 2 (100 mg, 0.44 mmol) instead of 1,3-dichloro-6,7-dihydroxy-5H-cyclopenta[c]pyridine-4-carbonitrile.

1H-NMR (500MHz, CDCl₃) δ 4.58 (q, J = 6.8 Hz, 1H), 4.42 (td, J = 8.5, 4.6 Hz, 1H), 4.09-3.94 (m, 3H), 3.89 (dd, J= 11.3, 4.9 Hz, 2H), 2.80 (dd, J= 15.4, 6.9 Hz, 2H), 2.73 (d, J = 7.9 Hz, 2H), 2.63-2.49 (m, 2H), 2.44-2.25 (m, 4H), 2.26-2.12 (m, 1H), 1.98 (t, J = 9.5 Hz, 1H), 1.77 (t, J = 6.4 Hz, 2H), 1.71-1.57 (m, 2H), 1.52 (dd, J = 8.8, 5.5 Hz, 1H), 1.48 (d, J = 6.1 Hz, 3H)

### [Example 3]

### 2-(3-(4-cyano-3-((S)-2-methylazetidin-1-yl)-6,7,8,9-tetrahydro-5H-cyclohepta[c]pyridin-1-yl)-3-azabicyclo[3.1.1]heptan-6-yl)acetic acid

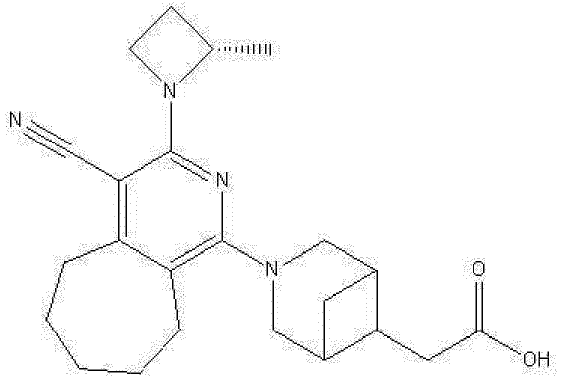

By the method described in Example 1, 2-(3-(4-cyano-3-((S)-2-methylazetidin-1-yl)-6,7,8,9-tetrahydro-5H-cyclohepta[c]pyridin-1-yl)-3-azabicyclo[3.1.1]heptan-6-yl)acetic acid (9.4 mg, 0.05 mmol, 24%, 3 steps) was obtained using the 1,3-dichloro-6,7,8,9-tetrahydro-5H-cyclohepta[c]pyridine-4-carbonitrile obtained in Preparation Example 3 (80 mg, 0.21 mmol) instead of 1,3-dichloro-6,7-dihydroxy-5H-cyclopenta[c]pyridine-4-carbonitrile.

1H-NMR (400MHz, CDCl₃) δ 4.56 (q, J = 6.9 Hz, 1H), 4.41 (td, J = 8.7, 4.6 Hz, 1H), 4.08-3.92 (m, 2H), 3.82 (dd, J= 16.9, 11.9 Hz, 2H), 3.71 (d, J= 11.4 Hz, 1H), 2.87 (dd, J = 11.4, 6.9 Hz, 2H), 2.69 (d, J = 7.8 Hz, 2H), 2.64 (q, J = 5.9 Hz, 2H), 2.43-2.31 (m, 1H), 2.30-2.22 (m, 3H), 2.17 (dd, J = 13.3, 8.2 Hz, 1H), 2.04-1.90 (m, 1H), 1.79 (t, J = 3.0 Hz, 2H), 1.74-1.50 (m, 5H), 1.44 (d, J = 5.9 Hz, 3H)

### [Example 4]

### 2-((1R,SS,6S)-3-(5-cyano-6-((S)-2-methylazetidin-1-yl)-3,4-dihydro-1H-pyrano[3,4-c]pyridin-8-yl)-3-azabicyclo[3.1.1]heptan-6-yl)acetic acid

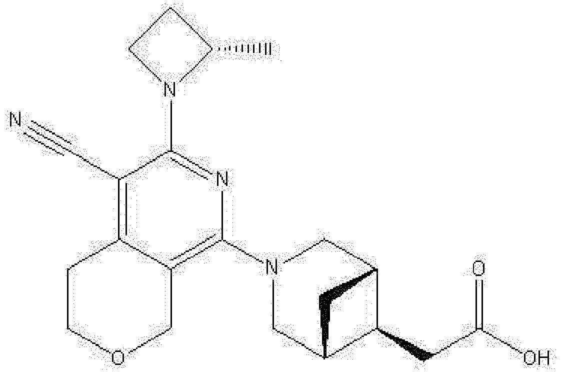

By the method described in Example 1, 2-((1R,5S,6S)-3-(5-cyano-6-((S)-2-methylazetidin-1-yl)-3,4-dihydro-1H-pyrano[3,4-c]pyridin-8-yl)-3-azabicyclo[3.1.1]heptan-6-yl)acetic acid (11 mg, 0.029 mmol, 7%, 3 steps) was obtained using the 6,8-dichloro-3,4-dihydro-1H-pyrano[3,4-c]pyridine-5-carbonitrile obtained in Preparation Example 4 (100 mg, 0.44 mmol) instead of 1,3-dichloro-6,7-dihydroxy-5H-cyclopenta[c]pyridine-4-carbonitrile.

1H-NMR (500MHz, CDCl₃) δ 4.71 (s, 2H), 4.60 (q, J = 6.8 Hz, 1H), 4.43 (td, J = 8.5, 4.8 Hz, 1H), 4.05 (q, J = 8.2 Hz, 2H), 4.00-3.77 (m, 8H), 2.88 (t, J = 5.8 Hz, 2H), 2.73 (d, J = 7.9 Hz, 2H), 2.48-2.26 (m, 5H), 2.15 (dd, J = 13.1, 7.6 Hz, 1H), 1.99 (t, J= 9.6 Hz, 1H), 1.56-1.39 (m, 5H)

### [Example 5]

### 2-(3-(7,7-diemthyl-2-((S)-2-methylazetidin-1-yl)-5,7-dihydrofuro [3,4-d]pyrimidin-4-yl)-3-azabicyclo[3.1.1]heptan-6-yl)acetic acid

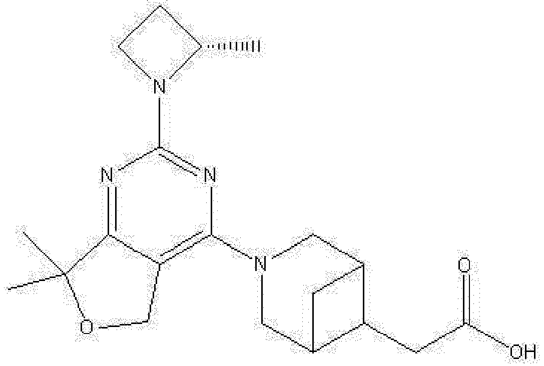

By the method described in Example 1, 2-(3-(7,7-dimethyl-2-((S)-2-methylazetidin-1-yl)-5,7-dihydrofuro[3,4-d]pyrimidin-4-yl)-3-azabicyclo[3.1.1]heptan-6-yl)acetic acid (6 mg, 0.016 mmol, 3%, 3 steps) was obtained using 2,4-dichloro-7,7-dimethyl-5,7-dihydrofuro[3,4-d]pyrimidine (refer to the synthesis method proposed in JMC, 54(9), 3426, 2011.; 130 mg, 0.59 mmol) instead of 1,3-dichloro-6,7-dihydroxy-5H-cyclopenta[c]pyridine-4-carbonitrile.

1H-NMR (500MHz, CDCl₃): δ 5.24 (d, J = 7.0 Hz, 2H), 4.49-4.36 (1H), 4.10-3.98 (m, 1H), 3.98-3.91 (m, 1H), 3.90-3.71 (m, 4H), 2.72 (d, J = 7.9 Hz, 1H), 2.65 (s, 0H), 2.58 (t, J= 5.5 Hz, 1H), 2.47-2.28 (m, 4H), 2.20-2.06 (m, 1H), 2.03-1.89 (m, 1H), 1.51 (d, J = 6.1 Hz, 3H), 1.48-1.37 (m, 7H)

### [Example 6]

### 2-((1R,SS,6S)-3-((S)-7-methyl-2-((S)-2-methylazetidin-1-yl)-5,7-dihydrofuro[3,4-d]pyrimidin-4-yl)-3-azabicyclo[3.1.1]heptan-6-yl)acetic acid

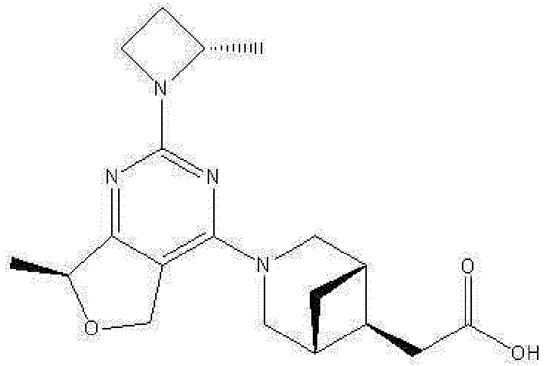

By the method described in Example 1, 2-((1R,5S,6S)-3-((S)-7-methyl-2-((S)-2-methylazetidin-1-yl)-5,7-dihydrofuro[3,4-d]pyrimidin-4-yl)-3-azabicyclo[3.1.1]heptan-6-yl)acetic acid (30 mg, 0.8 mmol, 26%, 3 steps) was obtained using the (S)-2,4-dichloro-7-methyl-5,7-dihydrofuro[3,4-d]pyrimidine obtained in Preparation Example 5 (63 mg, 0.31 mmol) instead of 1,3-dichloro-6,7-dihydroxy-5H-cyclopenta[c]pyridine-4-carbonitrile.

1H-NMR (400MHz, CDCl₃) δ 5.73-5.35 (m, 2H), 5.27 (ddd, J = 31.8, 10.1, 2.1 Hz, 2H), 5.10-4.92 (m, 1H), 4.45 (dd, J = 8.5, 5.7 Hz, 1H), 4.16-4.01 (1H), 4.01-3.92 (1H), 3.91-3.67 (m, 4H), 2.70 (d, J = 8.2 Hz, 2H), 2.45-2.27 (m, 4H), 2.24-2.06 (m, 1H), 2.04-1.89 (1H), 1.58-1.46 (3H), 1.42 (dd, J = 14.2, 5.9 Hz, 4H)

### [Example 7]

### 2-((1R,SS,6S)-3-((S)-7-methyl-2-((S)-2-methylazetidin-1-yl)-5,7-dihydrofuro[3,4-d]pyrimidin-4-yl)-3-azabicyclo[3.1.0]hexan-6-yl)acetic acid

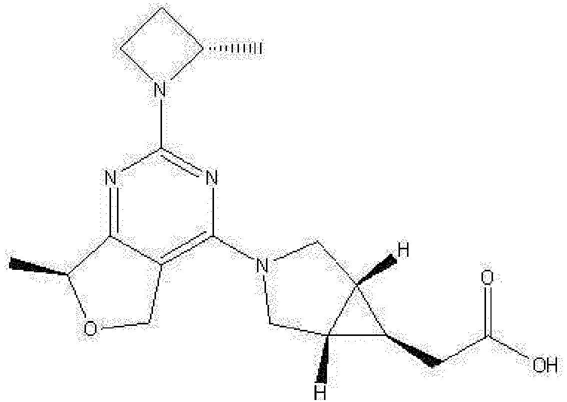

By the method described in Example 1, 2-((1R,5S,6S)-3-((S)-7-methyl-2-((S)-2-methylazetidin-1-yl)-5,7-dihydrofuro[3,4-d]pyrimidin-4-yl)-3-azabicyclo[3.1.0]hexan-6-yl)acetic acid (13 mg, 0.04 mmol, 11%, 3 steps) was obtained using the (S)-2,4-dichloro-7-methyl-5,7-dihydrofuro[3,4-d]pyrimidine obtained in Preparation Example 5 (78 mg, 0.38 mmol) instead of 1,3-dichloro-6,7-dihydroxy-5H-cyclopenta[c]pyridine-4-carbonitrile, and ethyl 2-((1R,5S,6S)-3-azabicyclo[3.1.0]hexan-6-yl)acetate (78 mg, 0.57 mmol) instead of methyl 2-(3-azabicyclo[3.1.1]heptan-6-yl)acetate hydrochloride.

1H-NMR (400MHz, CDCl₃): δ 5.25-5.06 (m, 2H), 4.97 (t, J = 6.3 Hz, 1H), 4.48-4.34 (m, 1H), 4.02 (td, J = 8.6, 4.5 Hz, 1H), 3.94 (q, J = 7.9 Hz, 1H), 3.81 (d, J = 25.3 Hz, 2H), 3.65-3.48 (m, 2H), 2.44-2.28 (m, 3H), 1.96 (t, J = 9.0 Hz, 1H), 1.60-1.48 (m, 5H), 1.43 (d, J = 6.4 Hz, 3H), 1.01 (d, J = 2.7 Hz, 1H)

### [Example 8]

### 2-((1R,SS,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)acetic acid

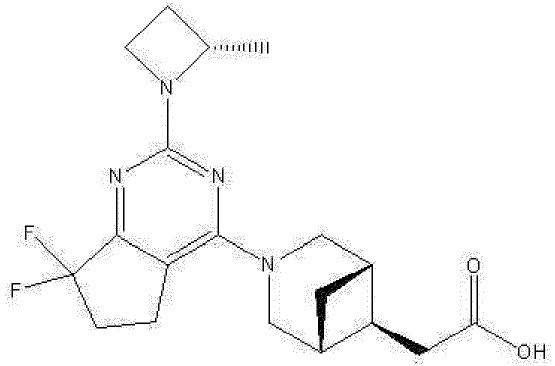

A compound of Example 8 was prepared through the following Steps A, B, C and D.

### (Step A) Preparation of ethyl 2-(3-(7,7-difluoro-2-(methylthio)-6,7-dihydro-5H-cyclopenta[d]pyrimidin-4-yl)-3-azabicyclo[3.1.1]heptan-6-yl)acetate

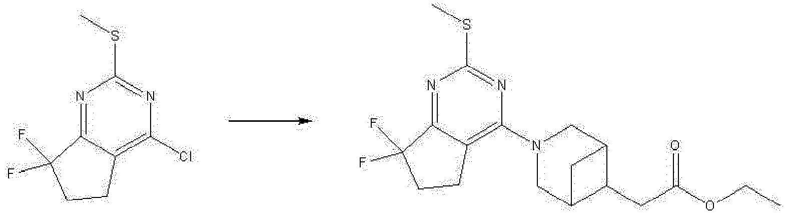

The 4-chloro-7,7-difluoro-2-(methylthio)-6,7-dihydro-5H-cyclopenta[d]pyrimidine obtained in Preparation Example 6 (130 mg, 0.55 mmol), ethyl 2-(3-azabicyclo[3.1.1]heptan-6-yl)acetate hydrochloride (121 mg, 0.55 mmol) and diisopropylethyl amine (0.29 ml, 1.65 mmol) were dissolved in dioxane (10 ml), and stirred at 50 °C for 8 hours. After the reaction was completed, an aqueous 1N hydrochloric solution was added, and extraction was performed with acetate. An organic layer was washed with an aqueous sodium chloride solution, an aqueous sodium hydrogen carbonate solution and water, dried with anhydrous magnesium sulfate, and filtered. The filtrate was concentrated under reduced pressure and purified by column chromatography (ethyl acetate/hexane = 1/3), thereby obtaining ethyl 2-(3-(7,7-difluoro-2-(methylthio)-6,7-dihydro-5H-cyclopenta[d]pyrimidin-4-yl)-3-azabicyclo[3.1.1]heptan-6-yl)acetate (170 mg, 0.44 mmol, 81%).

### (Step B) Preparation of ethyl 2-(3-(7,7-difluoro-2-(methylsulfonyl)-6,7-dihydro-5H-cyclopenta[d]pyrimidin-4-yl)-3-azabicyclo[3.1.1]heptan-6-yl)acetate

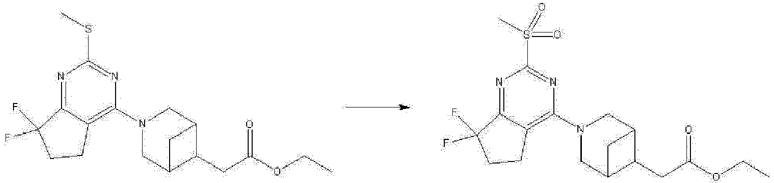

The ethyl 2-(3-(7,7-difluoro-2-(methylthio)-6,7-dihydro-5H-cyclopenta[d]pyrimidin-4-yl)-3-azabicyclo[3.1.1]heptan-6-yl)acetate (170 mg, 0.44 mmol) obtained in Step A and 3-chlorobenzoperoxy acid (248 mg, 1.11 mmol) were dissolved in dichloromethane (50 ml), and stirred at room temperature for 3 hours. After the reaction was completed, an aqueous sodium hydrogen carbonate solution was added, and extraction was performed with dichloromethane. An organic layer was washed with an aqueous sodium chloride solution, dried with anhydrous magnesium sulfate, and filtered. The filtrate was concentrated under reduced pressure, and purified by column chromatography (ethyl acetate/hexane = 1/2), thereby obtaining ethyl 2-(3-(7,7-difluoro-2-(methylsulfonyl)-6,7-dihydro-5H-cyclopenta[d]pyrimidin-4-yl)-3-azabicyclo[3.1.1]heptan-6-yl)acetate (180 mg, 0.43 mmol, 98%).

### (Step C) Preparation of ethyl 2-(3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)acetate

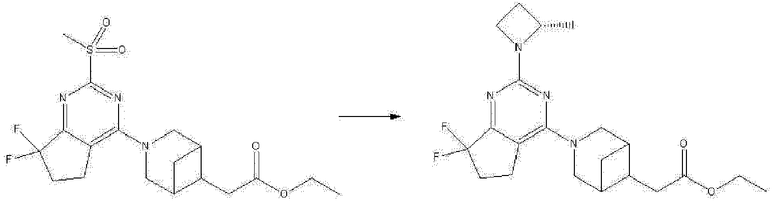

The ethyl 2-(3-(7,7-difluoro-2-(methylsulfonyl)-6,7-dihydro-5H-cyclopenta[d]pyrimidin-4-yl)-3-azabicyclo[3.1.1]heptan-6-yl)acetate obtained in Step B (180 mg, 0.43 mmol), (S)-2-methylazetidine hydrochloride (70 mg, 0.65 mmol) and potassium carbonate (180 mg, 1.30 mmol) were dissolved in N-methylpyrrolidine (15 ml), and stirred at 120 °C for 4 hours. After the reaction was completed, an aqueous 1N hydrochloric solution was added, and extraction was performed with acetate. An organic layer was washed with an aqueous sodium chloride solution, an aqueous sodium hydrogen carbonate solution and water, dried with anhydrous magnesium sulfate and filtered. The filtrate was concentrated under reduced pressure, and purified by column chromatography (ethyl acetate/hexane = 1/2), thereby obtaining ethyl 2-(3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)acetate (60 mg, 0.15 mmol, 34%).

### (Step D) Preparation of 2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)acetic acid

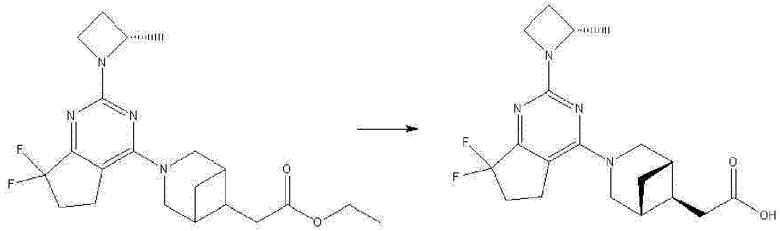

The ethyl 2-(3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)acetate obtained in Step C (60 mg, 0.15 mmol) and lithium hydroxide monohydrate (27 mg, 0.64 mmol) were dissolved in a 20% tetrahydrofuran solution (5 ml), and stirred at room temperature for 10 hours. After the reaction was completed, an aqueous 1N hydrochloric acid solution was added, followed by extraction with acetate. An organic layer was washed with an aqueous sodium chloride solution, dried with anhydrous magnesium sulfate and filtered. The filtrate was concentrated under reduced pressured, and purified by column chromatography (ethyl acetate/hexane = 1/2), thereby obtaining 2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)acetic acid (25 mg, 0.066 mmol, 45%, relatively more polar compound) and 2-((1R,5S,6R)-3-(7,7-difluoro-2-((S)-2-methylazetidin-1-yl)-6,7-dihydro-SH-cyclopenta[d]pyrimidin-4-yl)-3-azabicyclo[3.1.1]heptan-6-yl)acetic acid (relatively less polar compound).

1H-NMR (400MHz, CDCl₃) δ 4.42 (q, J = 6.7 Hz, 1H), 4.15-3.76 (m, 6H), 3.14 (q, J = 4.1 Hz, 2H), 2.70 (d, J = 7.8 Hz, 2H), 2.52-2.25 (6H), 2.11 (dd, J = 13.0, 8.0 Hz, 1H), 1.97-1.83 (m, 1H), 1.48 (d, J = 6.4 Hz, 3H), 1.41 (q, J = 4.9 Hz, 1H)

### [Example 9]

### 2-((1R,SS,6S)-3-(7,7-difluoro-2-((S)-2-methylazetidin-1-yl)-6,7-dihydro-5H-cyclopenta [d] pyrim idin-4-yl)-3-azabicyclo [3.1.0] hexan-6-yl)acetic acid

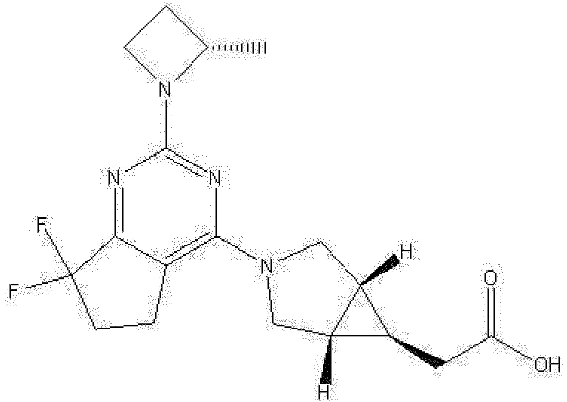

A compound of Example 9 was prepared through the following Steps A, B, C and D.

### (Step A) Preparation of 4-chloro-7,7-difluoro-2-(methylsulfonyl)-6,7-dihydro-5H-cyclopenta[d]pyrimidine

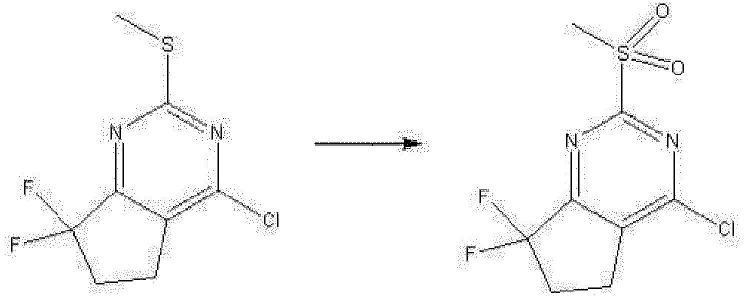

The 4-chloro-7,7-difluoro-2-(methylthio)-6,7-dihydro-5H-cyclopenta[d]pyrimidine obtained in Preparation Example 6 (1.67 g, 6.97 mmol) was dissolved in dichloromethane, and 70 wt% mCPBA (4.30 g, 17.43 mmol) was added, followed by stirring at room temperature for 2 hours. After the reaction was completed, a saturated sodium metabisulfite solution was added and stirred for 30 minutes, saturated sodium bicarbonate was added, followed by extraction with dichloromethane. An organic layer was washed with an aqueous sodium chloride solution, dried with anhydrous magnesium sulfate and filtered for concentration. The compound obtained after concentration (1.74 g, 6.48 mmol) was used in a subsequent reaction without a separate purification process.

1H-NMR (400 MHz, CDCl₃) δ3.45 (s, 3H), 3.20 (m, 2H), 2.75-2.86 (m, 2H)

### (Step B) Preparation of ethyl 2-((1R,5S,6S-3-(7,7-difluoro-2-(methylsulfonyl)-6,7-dihydro-5H-cyclopenta[d]pyrimidin-4-yl)-3-azabicyclo[3.1.0]hexan-6-yl)acetate

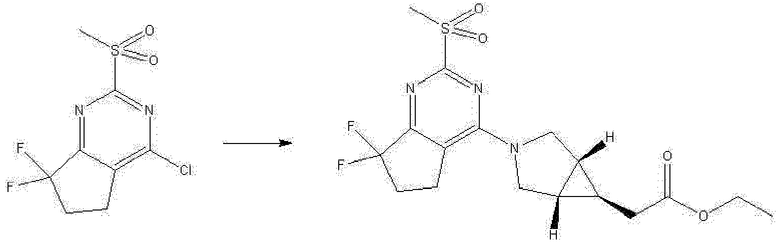

The compound obtained in Step A (1.74 g, 6.48 mmol) was dissolved in 1,4-dioxane, ethyl 2-((1R,5S,6S)-3-azabicyclo[3.1.0]hexan-6-yl)acetate hydrochloride (1.6 g, 7.78 mmol) was added to the 1,4-dioxane solution, and DIPEA (2.26 ml, 12.97 mmol) was added. The reactant was stirred at room temperature for 18 hours. After the reaction was completed, concentration under reduced pressure was performed. The concentrated compound was mixed with ethyl acetate and a saturate ammonium chloride solution for extraction. An organic layer was washed with a sodium chloride solution, dried with anhydrous magnesium sulfate, and filtered, followed by concentration under reduced pressure. The concentrated compound was purified by column chromatography (heptane/ethyl acetate = 1/1), thereby obtaining ethyl 2-((1R,5S,6S-3-(7,7-difluoro-2-(methylsulfonyl)-6,7-dihydro-5H-cyclopenta[d]pyrimidin-4-yl)-3-azabicyclo[3.1.0]hexan-6-yl)acetate (1.327 g, 51%).

1H-NMR (400MHz, CDCl₃) δ 4.16 (m, 4H), 3.73-4.02 (m, 2H), 3.30-3.40 (m, 3H), 3.22-3.30 (m, 2H), 2.57-2.68 (m, 2H), 2.24-2.41 (m, 2H), 1.65 (s, 2H), 1.26-1.33 (m, 3H), 0.95-1.01 (m, 1H)

### (Step C) Preparation of ethyl 2-((1R,5S,6S)-3-(7,7-difluoro-2-((S)-2-methylazetidin-1-yl)-6,7-dihydro-SH-cyclopenta[d]pyrimidin-4-yl)-3-azabicyclo[3.1.0]hexan-6-yl)acetate

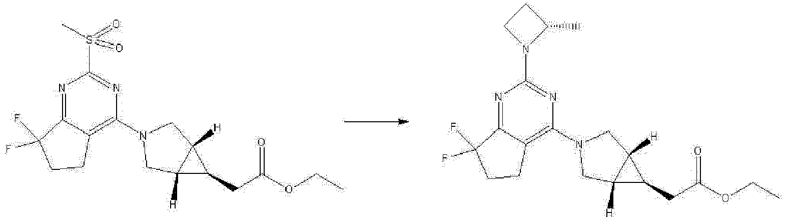

The compound obtained in Step B (1.32 g, 3.29 mmol) was dissolved in NMP, potassium carbonate (2.272 g, 16.44 mmol) was added, and (S)-methylazetidine hydrochloride was dissolved in NMP and then added thereto. A reactant was stirred at 120 °C for 4 hours. After the reaction was completed, the temperature was lowered to room temperature, an aqueous ammonium chloride solution and ethyl acetate were added, and stirred for several minutes, followed by performing extraction. An organic layer was washed with an aqueous ammonium chloride solution and an aqueous sodium chloride solution, dried with anhydrous magnesium sulfate, and filtered. The filtrate was concentrated under reduced pressure, and the concentrated compound was purified by column chromatography (heptane/ethyl acetate = 1/1), thereby obtaining ethyl 2-((1R,SS,6S)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)acetate (770 mg, 59%).

### (Step D) Preparation of 2-((1R,SS,6S)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)acetic acid

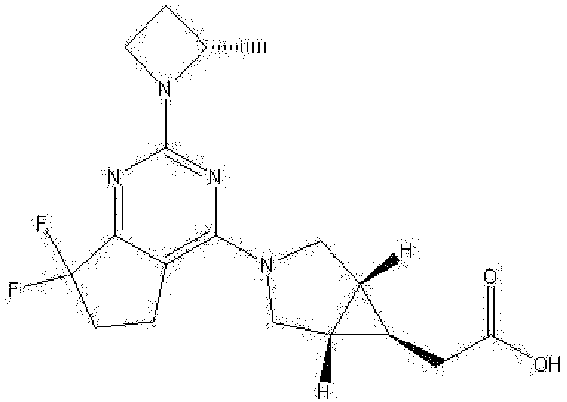

The compound obtained in Step C (305 mg, 0.77 mmol) was dissolved in tetrahydrofuran (8 ml), and lithium hydroxide monohydrate (65 mg, 1.55 mmol) was added. Water (4 ml) and methanol (2 ml) were added sequentially, and the reactants were stirred for 18 hours. After the reaction was completed, 1.5 ml of 1N HCl was added, and an aqueous ammonium chloride solution and ethyl acetate were added for extraction. An organic layer was washed with sodium chloride, dried with anhydrous magnesium sulfate and then filtered. The filtrate was concentrated under reduced pressure, and the concentrated compound was purified by column chromatography (MC/MeOH=10/1), thereby obtaining 2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)acetic acid (262 mg, 93%).

1H-NMR (400 MHz, CDCl₃) δ4.39 (dt, J = 20.4, 6.3 Hz, 1H), 4.10-3.86 (m, 4H), 3.71-3.52 (m, 2H), 3.46 (d, J = 16.9 Hz, 1H), 3.07-2.90 (m, 2H), 2.51-2.24 (m, 5H), 1.97-1.83 (m, 1H), 1.58-1.43 (m, 5H), 1.03-0.88 (m, 1H)

### [Example 10]

### 2-((1R,SS,6S)-3-(8,8-difluoro-2-((S)-2-methylazetidin-1-yl)-5,6,7,8-tetrahydroquinazolin-4-yl)-3-azabicyclo[3.1.1]heptan-6-yl)acetic acid

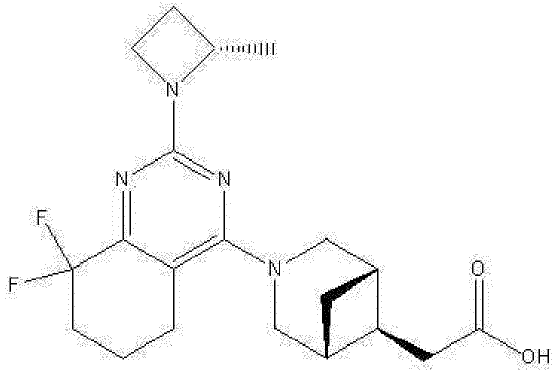

By the method described in Example 8, 2-((1R,5S,6S)-3-(8,8-difluoro-2-((S)-2-methylazetidin-1-yl)-5,6,7,8-tetrahydroquinazolin-4-yl)-3-azabicyclo[3.1.1]heptan-6-yl)acetic acid (18 mg, 0.046 mmol, 8%, 4 steps) and 2-((1R,5S,6R)-3-(8,8-difluoro-2-((S)-2-methylazetidin-1-yl)-5,6,7,8-tetrahydroquinazolin-4-yl)-3-azabicyclo[3.1.1]heptan-6-yl)acetic acid were obtained using the 4-chloro-8,8-difluoro-2-(methylthio)-5,6,7,8-tetrahydroquinazoline obtained in Preparation Example 7 (150 mg, 0.60 mmol).

1H-NMR (400MHz, CDCl₃) δ 5.07-4.47 (1H), 4.39 (t, J = 6.6 Hz, 1H), 4.12-3.99 (m, 2H), 3.94 (q, J = 8.1 Hz, 3H), 3.87 (d, J = 11.0 Hz, 1H), 2.84-2.58 (m, 4H), 2.31 (d, J = 8.2 Hz, 4H), 2.26-2.14 (m, 2H), 2.11 (d, J = 5.5 Hz, 1H), 1.91 (t, J = 8.9 Hz, 1H), 1.80 (t, J = 5.7 Hz, 2H), 1.47 (d, J = 5.9 Hz, 3H), 1.44-1.35 (m, 1H)

### [Example 11]

### 2-((1R,SS,6S)-3-(8,8-difluoro-2-((S)-2-methylazetidin-1-yl)-5,6,7,8-tetrahydroquinazolin-4-yl)-3-azabicyclo[3.1.0]hexan-6-yl)acetic acid

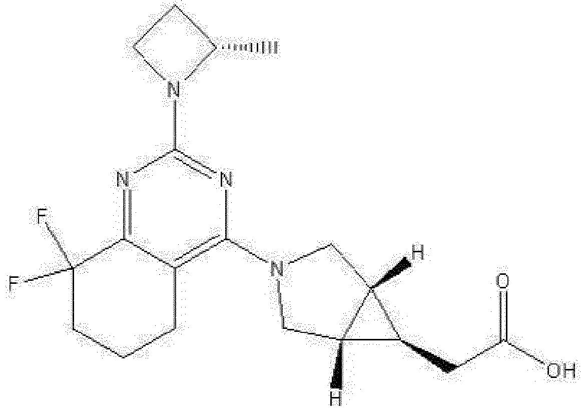

A compound of Example 11 was prepared through the following Steps A, B, C and D.

### (Step A) Preparation of 4-chloro-8,8-difluoro-2-(methylsulfonyl)-5,6,7,8-tetrahydroquinazoline

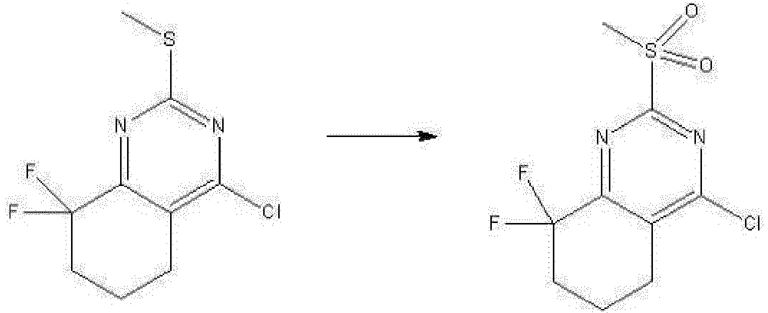

4-chloro-8,8-difluoro-2(methylthio)-5,6,7,8-tetrahydroquinazoline (500 mg, 2.00 mmol) and 3-chlorobenzoperoxy acid (862 mg, 5.00 mmol) were dissolved in dichloromethane (30 ml), and stirred at room temperature for 18 hours. After the reaction was completed, an aqueous sodium metabisulfite solution was added, and extraction was performed with dichloromethane. An organic layer was washed with an aqueous sodium bicarbonate solution, dried with anhydrous magnesium sulfate, and filtered. The filtrate was concentrated under reduced pressure, and purified by column chromatography (ethyl acetate/hexane = 1/2), thereby obtaining 4-chloro-8,8-difluoro-2-(methylsulfonyl)-5,6,7,8-tetrahydroquinazoline (527 mg, 1.86 mmol, 93%).

1H-NMR (500MHz, CDCl₃) δ3.41 (s, 3H), 3.03-2.89 (m, 2H), 2.52-2.33 (m, 2H), 2.22-2.07 (m, 2H)

### (Step B) Preparation of ethyl 2-((1R,5S,6S)-3-(8,8-difluoro-2-(methylsulfonyl)-5,6,7,8-tetrahydroquinazolin-4-yl)-3-azabicyclo[3.1.0]hexan-6-yl)acetate

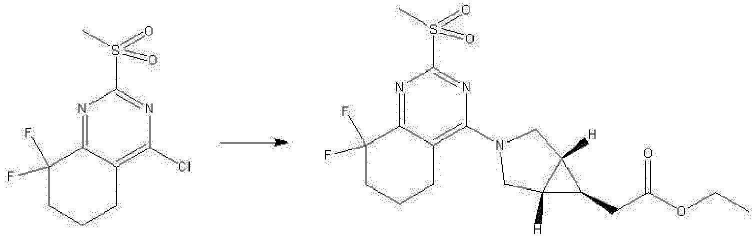

The compound obtained in Step A (2.03 g, 7.18 mmol) was dissolved in 1,4-dioxane, ethyl 2-((1R,5S,6S)-3-azabicyclo[3.1.0]hexan-6-yl)acetate hydrochloride (1.6 g, 7.78 mmol) was added to a 1,4-dioxane solution, DIPEA (2.5 ml, 14.36 mmol) was added, and a reactant was stirred at room temperature for 18 hours. After the reaction was completed, concentration under reduced pressure was performed. The concentrated compound was mixed with ethyl acetate and a saturated ammonium chloride solution for extraction. An organic layer was washed with a sodium chloride solution, anhydrous magnesium sulfate was added and filtered, followed by concentration under reduced pressure. The concentrated compound was purified by column chromatography (heptane/ethyl acetate = 1/1), thereby obtaining ethyl 2-((1R,5S,6S)-3-(8,8-difluoro-2-(methylsulfonyl)-5,6,7,8-tetrahydroquinazolin-4-yl)-3-azabicyclo[3.1.0]hexan-6-yl)acetate (2.360 g, 79%).

1H-NMR (400 MHz, CDCl₃) δ4.13-4.24 (m, 4H), 3.80-3.83 (m, 2H), 3.33 (s, 3H), 2.90 (m, 2H), 2.27-2.39 (m, 4H), 1.94-1.97 (m, 2H), 1.60-1.63 (m, 2H), 1.28 (t, J = 6.8Hz, 2H), 0.94-0.97 (m, 1H)

### (Step C) Preparation of ethyl 2-((1R,5S,6S)-3-(8,8-difluoro-2-((S)-2-methylazetidin-1-yl)-5,6,7,8-tetrahydroquinazolin-4-yl)-3-azabicyclo[3.1.0]hexan-6-yl)acetate

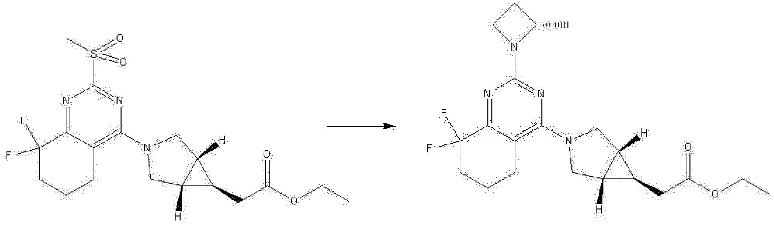

The compound obtained in Step B (2.36 g, 5.68 mmol) was dissolved in NMP (20 ml), and potassium carbonate (3.93 g, 28.4 mmol) was added. (S)-2-methylazetidine hydrochloride (1.22 g, 11.36 mmol) was dissolved in NMP (8 ml) and added, and the reactants were stirred at 120 °C for 4 hours. When the reaction was completed, the temperature was lowered to room temperature, and an aqueous ammonium chloride solution and ethyl acetate were added, and the resulting solution was stirred for several minutes for extraction. An organic layer was washed with an aqueous ammonium chloride solution and an aqueous sodium chloride solution, anhydrous magnesium sulfate was added for drying and then filtered. The filtrate was concentrated under reduced pressure, and the concentrated compound was purified by column chromatography (heptane/ethyl acetate = 1/1), thereby obtaining ethyl 2-((1R,5S,6S)-3-(8,8-difluoro-2-((S)-2-methylazetidin-1-yl)-5,6,7,8-tetrahydroquinazolin-4-yl)-3-azabicyclo[3.1.0]hexan-6-yl)acetate (974 mg, 42%).

1H-NMR (500 MHz, CDCl₃) δ4.39-4.44 (m, 1H), 4.15-4.20 (m, 2H), 4.02-4.07 (m, 2H), 3.92-3.99 (m, 2H), 3.56 (m, 2H), 2.65 (d, J = 5.8 Hz, 2H), 2.32-2.38 (m, 1H), 2.30 (d, J = 7.0 Hz, 2H), 2.20-2.26 (m, 2H), 1.92-1.98 (m, 1H), 1.82-1.87 (m, 2H), 1.50 (t, J = 6.4 Hz, 5H), 1.29 (t, J = 7.2 Hz, 3H)

### (Step D) Preparation of 2-((1R,5S,6S)-3-(8,8-difluoro-2-((S)-2-methylazetidin-1-yl)-5,6,7,8-tetrahydroquinazolin-4-yl)-3-azabicyclo[3.1.0]hexan-6-yl)acetic acid

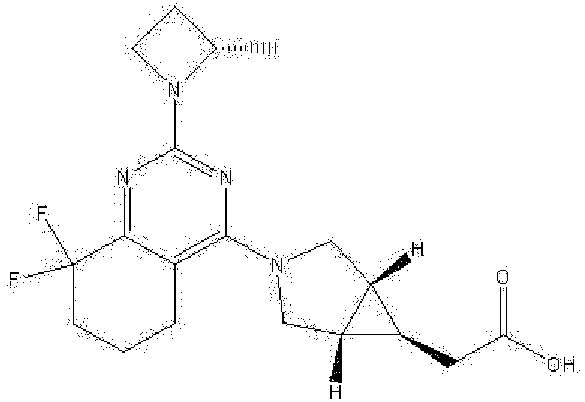

The compound obtained in Step C (221 mg, 0.544 mmol) was dissolved in tetrahydrofuran (6 ml), lithium hydroxide monohydrate (46 mg, 1.08 mmol) was added, and water (3 ml) and methanol (1.5 ml) were added, followed by stirring for 18 hours. After the reaction was completed, 1.0 ml of 1N HCl was added, and then an aqueous ammonium chloride solution and ethyl acetate were added for extraction. An organic layer was washed with sodium chloride, dried with anhydrous magnesium sulfate, and filtered. The filtrate was concentrated under reduced pressure, and the concentrated compound was purified by column chromatography (MC/MeOH=10/1), thereby obtaining 2-((1R,5S,6S)-3-(8,8-difluoro-2-((S)-2-methylazetidin-1-yl)-5,6,7,8-tetrahydroquinazolin-4-yl)-3-azabicyclo[3.1.0]hexan-6-yl)acetic acid (82 mg, 40%).

1H-NMR (400MHz, CDCl₃) δ 4.37 (dt, J = 20.4, 6.3 Hz, 1H), 4.07-3.86 (m, 4H), 3.62-3.46 (3H), 2.61 (t, J = 5.9 Hz, 2H), 2.38-2.27 (m, 3H), 2.27-2.13 (m, 2H), 1.97-1.86 (m, 1H), 1.86-1.75 (m, 2H), 1.52-1.43 (5H), 1.06-0.94 (1H)

### [Example 12]

### 2-((1R,SS,6S)-3-(8,8-difluoro-2-(pyrrolidin-1-yl)-5,6,7,8-tetrahydroquinazolin-4-yl)-3-azabicyclo[3.1.0]hexan-6-yl)acetic acid

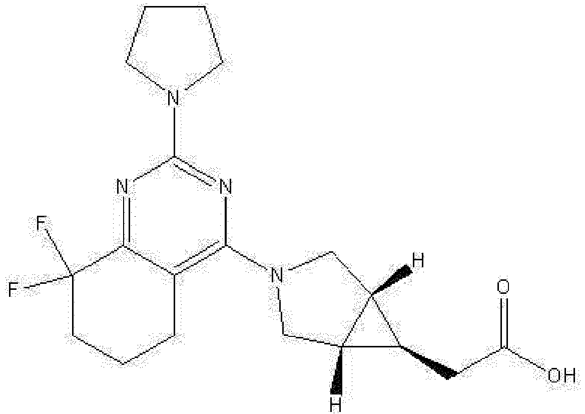

By the method described in Example 11, 2-((1R,5S,6S)-3-(8,8-difluoro-2-(pyrrolidin-1-yl)-5,6,7,8-tetrahydroquinazolin-4-yl)-3-azabicyclo[3.1.0]hexan-6-yl)acetic acid (17 mg, 0.044 mmol, 54%, 2 steps) was obtained using pyrrolidine instead of (S)-2-methylazetidine hydrochloride.

1H-NMR (400MHz, CDCl₃) δ 4.01(d, J=11.0Hz, 2H), 3.54(m, 6H), 3.48(s, 1H), 2.61(m, 2H), 2.33(d, J=7.3Hz, 2H), 2.22(m, 2H), 1.91(m, 4H), 1.80-1.85(m, 2H), 1.48(s, 2H), 0.98-1.03(m, 1H)

### [Experimental Example] Measurement of in vitro activity of KHK inhibitor

KHK was expressed in *E. coli,* and purified using a His tag. To measure the activity of the purified KHK, a Transcreener ADP2 TR-FRET Red Assay kit of BellBrook Labs was used. The Transcreener ADP2 TR-FRET Red Assay kit is a method of measuring an amount of generated ADP using TR-FRET after proper concentrations of KHK and ATP, and a fructose-containing solution were reacted for 15 minutes. To measure the activity of a KHK inhibitor, KHK and a proper concentration of an inhibitor were first reacted for 30 minutes, and reacted with a solution containing a KHK substrate for 15 minutes. In addition, after the TR-FRET reaction for 1 hour, fluorescence was measured using an Envision apparatus of PerkinElmer. A setting value of the Envision apparatus was determined according to the TR-FRET optimization procedures published by BellBrook Labs.

The resulting value for each concentration of the KHK inhibitor is determined a ratio of a 665-nm wavelength and a 615-nm wavelength. Here, the 665/615 ratio was converted to an ADP concentration based on an ADP standard curve, and the inhibitory activity, a Ki value, was obtained using the Morrison equation in statistical software (Prizm).

The Ki values of the example compounds obtained through the experiment are shown in Table 1.

**[Table 1]**

| | Example 1 | Example 2 | Example 3 | Example 4 | Example 5 | Example 6 |
|---|---|---|---|---|---|---|
| Ki | D | D | D | D | C | C |

| | Example 7 | Example 8 | Example 9 | Example 10 | Example 11 | Example 12 |
|---|---|---|---|---|---|---|
| Ki | C | D | D | D | D | C |

(A≥10 µM, B≥1 µM, C= 1∼0.1 µM, D≤ 0.1 µM)

The Ki value is a value that indicates the binding affinity between an inhibitor and an enzyme, and the lower the Ki value, the higher the inhibitory activity. Accordingly, from the result, it can be confirmed that the compounds of the examples of the present invention exhibit excellent inhibitory activity.

### [Industrial Applicability]

The 3-azabicycloalkyl derivative compound of Formula 1 according to the present invention has KHK inhibitory activity, and can be effectively used in preventing or treating a metabolic disease such as diabetes, complications of diabetes, obesity, non-alcoholic steatohepatitis or steatohepatitis.

## Claims

1. A compound of Formula 1 below, or a pharmaceutically acceptable salt or isomer thereof. 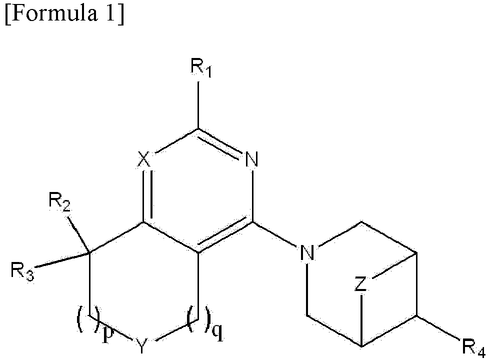 In Formula 1,
R₁ is unsubstituted or C₁-C₅ alkyl-substituted C₃-C₇ heterocycloalkyl having one to three N atoms,
R₂ and R₃ are each independently hydrogen, halogen or C₁-C₅ alkyl,
R₄ is -(CH₂)ₘCO₂H,
m, p and q are each independently an integer of 0 to 2,
X is N or C-CN,
Y is CH₂ or O, and
Z is CH₂ or a single bond.

2. The compound of claim 1, wherein the compound is a compound selected from the following group, or a pharmaceutically acceptable salt or isomer thereof:
2-((1R,5S,6S)-3-(4-cyano-3-((S)-2-methylazetidin-1-yl)-6,7-dihydro-5H-cyclopenta[c]pyridin-1-yl)-3-azabicyclo[3.1.1]heptan-6-yl)acetic acid;
2-((1R,5S,6S)-3-(4-cyano-3-((S)-2-methylazetidin-1-yl)-5,6,7,8-tetrahydroisoquinolin-1-yl)-3-azabicyclo[3.1.1]heptan-6-yl)acetic acid;
2-(3-(4-cyano-3-((S)-2-methylazetidin-1-yl)-6,7,8,9-tetrahydro-5H-cyclohepta[c]pyridin-1-yl)-3-azabicyclo[3.1.1]heptan-6-yl)acetic acid;
2-((1R,5S,6S)-3-(5-cyano-6-((S)-2-methylazetidin-1-yl)-3,4-dihydro-1H-pyrano[3,4-c]pyridin-8-yl)-3-azabicyclo[3.1.1]heptan-6-yl)acetic acid;
2-(3-(7,7-dimethyl-2-((S)-2-methylazetidin-1-yl)-5,7-dihydrofuro[3,4-d]pyrimidin-4-yl)-3-azabicyclo[3.1.1]heptan-6-yl)acetic acid;
2-((1R,5 S,6S)-3-((S)-7-methyl-2-((S)-2-methylazetidin-1-yl)-5,7-dihydrofuro[3,4-d]pyrimidin-4-yl)-3-azabicyclo[3.1.1]heptan-6-yl)acetic acid;
2-((1R,5 S,6S)-3-((S)-7-methyl-2-((S)-2-methylazetidin-1-yl)-5,7-dihydrofuro[3,4-d]pyrimidin-4-yl)-3-azabicyclo[3.1.0]hexan-6-yl)acetic acid;
2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.1]heptan-6-yl)acetic acid;
2-((1R,5S,6S)-3-(7,7-difluoro-2-((S)-2-methylazetidin-1-yl)-6,7-dihydro-5H-cyclopenta[d]pyrimidin-4-yl)-3-azabicyclo[3.1.0]hexan-6-yl)acetic acid;
2-((1R,5S,6S)-3-(8,8-difluoro-2-((S)-2-methylazetidin-1-yl)-5,6,7,8-tetrahydroquinazolin-4-yl)-3-azabicyclo[3.1.1]heptan-6-yl)acetic acid;
2-((1R,5S,6S)-3-(8,8-difluoro-2-((S)-2-methylazetidin-1-yl)-5,6,7,8-tetrahydroquinazolin-4-yl)-3-azabicyclo[3.1.0]hexan-6-yl)acetic acid; and
2-((1R,5S,6S)-3-(8,8-difluoro-2-(pyrrolidin-1-yl)-5,6,7,8-tetrahydroquinazolin-4-yl)-3-azabicyclo[3.1.0]hexan-6-yl)acetic acid.

3. The compound of claim 1 or 2, wherein the compound, or a pharmaceutically acceptable salt or isomer thereof is used as a ketohexokinase (KHK) inhibitor.

4. The compound of claim 1 or 2, wherein the compound, or a pharmaceutically acceptable salt or isomer thereof is used in prevention or treatment of a metabolic disease.

5. A pharmaceutical composition for inhibiting ketohexokinase (KHK), comprising:
the compound according to claim 1 to 2, or a pharmaceutically acceptable salt or isomer thereof; and a pharmaceutically acceptable carrier.

6. A pharmaceutical composition for preventing or treating a ketohexokinase (KHK)-related metabolic disease, comprising:
the compound according to claim 1 or 2, or a pharmaceutically acceptable salt or isomer thereof; and a pharmaceutically acceptable carrier.

7. The pharmaceutical composition of claim 6, wherein the metabolic disease is selected from the group consisting of diabetes, complications of diabetes, obesity, non-alcoholic steatohepatitis, and steatohepatitis.

8. A method of preparing a pharmaceutical composition for preventing or treating a ketohexokinase (KHK)-related metabolic disease, comprising:
mixing the compound according to claim 1 or 2, or a pharmaceutically acceptable salt or isomer thereof as an active ingredient, with a pharmaceutically acceptable carrier.

9. The method of claim 8, wherein the metabolic disease is selected from the group consisting of diabetes, complications of diabetes, obesity, non-alcoholic steatohepatitis, and steatohepatitis.
